# EUROPEAN PATENT SPECIFICATION

(11) **EP 3 565 889 B1**
(45) Date of publication and mention of the grant of the patent: **26.03.2025**
(21) Application number: 18736114.2
(22) Date of filing: 05.01.2018
(51) Int. Cl.: A61K 39/00, A61P 35/00, C07K 14/705, C12N 5/0783

(54) **GENETICALLY MODIFIED NK-92 CELLS WITH DECREASED CD96/TIGIT EXPRESSION**
GENETISCH MODIFIZIERTE NK-92-ZELLEN MIT VERMINDERTER CD96/TIGIT-EXPRESSION
CELLULES NK-92 GÉNÉTIQUEMENT MODIFIÉES À EXPRESSION RÉDUITE DE CD96/TIGIT

(30) Priority: 06.01.2017 US 201762443621 P; 16.02.2017 US 201762459873 P; 16.02.2017 US 201762459877 P
(43) Date of publication of application: 13.11.2019
(73) Proprietor: ImmunityBio, Inc., Culver City, CA 90232 (US)
(72) Inventor: NAVARRO, Francisco, Culver City California 90232 (US); KLINGEMANN, Hans, Culver City California 90232 (US)
(74) Representative: Elkington and Fife LLP
(86) International application number: PCT/US2018/012624
(87) International publication number: WO 2018/129346

(56) References cited:
- WO-A1-2015/024042
- WO-A1-2016/176756
- WO-A2-2016/191643
- US-A1- 2016 067 356
- FURUTANI ELISSA ET AL: "siRNA Inactivation of the Inhibitory Receptor NKG2A Augments the Anti-Tumor Effects of Adoptively Transferred NK Cells In Tumor-Bearing Hosts", BLOOD, AMERICAN SOCIETY OF HEMATOLOGY, US, vol. 116, no. 21, 19 November 2010 (2010-11-19), pages 1015, XP086606187, ISSN: 0006-4971, DOI: 10.1182/BLOOD.V116.21.1015.1015
- OLIVER HÖLSKEN ET AL: "Exploiting natural killer cells for therapy of melanoma", J DTSCH DERMATOL GES, WILEY-BLACKWELL VERLAG GMBH, DE, vol. 13, no. 1, 1 January 2015 (2015-01-01), pages 23 - 28, XP002792073, ISSN: 1610-0387, [retrieved on 20150106], DOI: 10.1111/DDG.12557
- BLAKE, STEPHEN J. ET AL.: "Molecular pathways: targeting CD 96 and TIGIT for cancer immunotherapy", CLINICAL CANCER RESEARCH, vol. 22, no. 21, 1 November 2016 (2016-11-01), pages OF1 - OF6, XP055419815
- CARLSTEN, MATTIAS ET AL.: "Genetic manipulation of NK cells for cancer immunotherapy: techniques and clinical implications", FRONTIERS IN IMMUNOLOGY, vol. 6, no. 266, June 2015 (2015-06-01), pages 1 - 9, XP055248039
- PENG, YUN-PENG ET AL.: "Altered expression of CD 226 and CD 96 on natural killer cells in patients with pancreatic cancer", ONCOTARGET, vol. 7, no. 41, 11 October 2016 (2016-10-11), pages 66586 - 66594, XP055512450

## Description

### BACKGROUND OF THE INVENTION

Cell-based immunotherapies are a powerful tool for the treatment of cancer. Early success in the treatment of patients with lymphoid malignancies, using engineered primary T cells expressing chimeric antigen receptors (CAR-T cells), has shown great promise for cancer treatment. Immunotherpaies based on the use of natural killer (NK) cells are also being developed, although the the field of NK cell-based immunotherapies is not as advanced as that employing T cells.

NK-92 is a cytolytic cancer cell line which was discovered in the blood of a subject suffering from a non-Hodgkins lymphoma and then immortalized *ex vivo.* NK-92 cells are derived from NK cells, but lack the major inhibitory receptors that are displayed by normal NK cells, while retaining the majority of the activating receptors. NK-92 cells do not, however, attack normal cells nor do they elicit an unacceptable immune rejection response in humans. The NK-92 cell line is characterized, *e.g.,* in WO1998/49268 and U.S. Patent Application No. 20020068044.

As noted above, a unique feature of activated NK-92 (aNK) cells is that they express multiple activating receptors (NKp30, NKp46, 2B4, NKGD, E, CD28, CD226), but lack most of the currently known inhibitory KIR receptors (Maki et al., J Hematother Stem Cell Res. 10:369-83, 2001). This unique phenotype may account for the broad anti-tumor activity of aNK cells. The activating receptor CD226, also known as DNAM1, belongs to a family of receptors that bind to nectin and nectin-like family proteins and has a crucial role in controlling NK cell-mediated cytotoxicity (Shibuya et al., Immunity. 4:573-81, 1996). This family also includes the inhibitory receptors CD96 and TIGIT (Martinet et al., Nat Rev Immunol. 15:243-54, 2015), which are also expressed by aNK cells. All three receptors share a common ligand, CD155 (also known as PVR, polio virus receptor), to which they bind with different affinities (Martinet *et al., supra*)*.* CD155 expression is frequently upregulated in tumor cells, and its over-expression is associated with cancer invasiveness and metastasis (Hirota et al., Oncogene 24:2229-35, 2005; Sloan et al., BMC Cancer 4:73, 2004.). CD96 and TIGIT have also been implicated as inhibitory immune checkpoints in NK cells (Chan et al., Nat Immunol. 15:431-8, 2014).
Blake et al (Clin Cancer Res. 2016 Nov 1;22(21):5183-5188) note that the receptors CD96 and TIGIT are expressed on the surface of T and natural killer (NK) cells, and recent studies suggest both play important inhibitory roles in immune function. The authors review the rationale behind targeting CD96 and TIGIT, and discuss the potential approaches in translating these preclinical findings into novel clinical agents. Holsken et al (JDDG, Volume 13, Issue1 January 2015: pages 23-28) review several approaches aimed at harnessing NK cells to treat melanoma patients and to counteract existing tumor escape mechanisms, and summarize the most recent advances in the field.

NK-92 cells have also been evaluated as a potential therapeutic agent in the treatment of certain cancers. This invention provides advances in NK-92-based therapies to treat cancers.

### BRIEF SUMMARY OF ASPECTS OF THE INVENTION

The invention is defined by the claims. The invention provides a modified NK-92 cell comprising one or more alterations that inhibit expression of one or more target genes, wherein the CD96 is one of the one or more target genes. In some embodiments, TIGIT is targeted in addition to CD96.

In one aspect, the disclosure provides a CD96-modified NK-92 cell that comprises a modification that inhibits expression of CD96. In some embodiments, the CD96-modified NK-92 cells comprise an interfering RNA that targets CD96 and inhibits its expression. In some embodiments, the amount of CD96 expressed by the CD96-modified NK-92 cell is decreased by at least 20%, at least 30%, at least 50%, at least 60%, or at least 80%, or greater, compared to NK-92 cells that do not have the CD96-targeted alteration. In some embodiments, the CD96-modified NK-92 cell is produced by knocking down or knocking out in an NK-92 cell.

In some embodiments, the CD96-modified NK-92 cell expresses at least one Fc receptor, or at least one chimeric antigen receptor (CAR), or both at least one Fc receptor and at least one CAR on the cell surface. In some embodiments, the at least one Fc receptor is CD16 or a CD16 polypeptide having a valine at position 176 (as numbered with reference to the precursor full-length human CD16, including the N-terminal methionine as position 1). In some embodiments, the at least one Fc receptor comprises a polynucleotide sequence encoding a polypeptide having at least 90% sequence identity to the amino acid sequence of SEQ ID NO: 13 and comprises a valine at position 176. In some embodiments, the at least one Fc receptor is FcγRIII. In some embodiments, the CAR comprises a cytoplasmic domain of FcεRIγ. In some embodiments, the CAR targets a tumor-associated antigen. In some embodiments, the CD96-modified NK-92 cell further expresses a cytokine. In some embodiments, the cytokine is interleukin-2 or a variant thereof. In some embodiments, the cytokine is targeted to the endoplasmic reticulum.

In another aspect, the disclosure provides a method for producing an NK-92 cell that expresses decreased levels of CD96 relative to a control NK-92 cell, the method comprising genetically modifying CD96 expression in the NK-92 cell. In some embodiments, the step of genetically modifying CD96 expression comprises contacting an NK-92 cell to be modified with an interfering RNA targeting CD96. In some embodiments, the interfering RNA targeting CD96 is an siRNA, an shRNA, a microRNA, or a single stranded interfering RNA.

In some embodiments, the step of genetically modifying CD96 expression comprises modifying the CD96 gene with a zinc finger nuclease (ZFN), a Tale-effector domain nuclease (TALEN), or a CRIPSR/Cas system. In some embodiments, genetically modifying CD96 gene expression comprises: i) introducing a clustered regularly interspaced short palindromic repeat-associated (Cas) protein into the NK-92 cell and ii) introducing one or more ribonucleic acids in the NK-92 cell to be modified, wherein the ribonucleic acids direct the Cas protein to hybridize to a target motif of a CD96 gene sequence, and wherein the target motif is cleaved. In some embodiments, the Cas protein is introduced into the NK-92 cell in protein form. In some embodiments, the Cas protein is introduced into the NK-92 cell by introducing a Cas nucleic acid coding sequence. In some embodiments, the Cas protein is Cas9. In some embodiments, the target motif is a 20-nucleotide DNA sequence. In some embodiments, the target motif is in the second exon of the CD96 gene sequence (Accession Number NM_198196 transcript variant 1), which is shared by CD96 transcript variant 2 (Accession Number NM_005816) and CD96 transcript variant 3 (Accession Number NM_001318889). In some embodiments, the one or more ribonucleic acids are selected from the group consisting of SEQ ID NOs. 1-4.

In a further aspect, provided herein is a composition comprising a plurality of the CD96-modified NK-92 cells, *e.g.,* as described above. In some embodiments, the composition also comprises a physiologically acceptable excipient.

In an additional aspect, provided herein is a modified NK-92 cell line comprising a plurality of any of the CD96-modified NK-92 cells described herein, *e.g.,* in the preceiding paragraphs. In some embodiments, the cells of the cell line undergo less than 10 population doublings. In some embodiments, the cells of the cell line are cultured in media containing less than 10 U/ml of IL-2.

In another aspect, provided herein is a cell for use in a method of treating cancer in a patient in need thereof, the method comprising administering to the patient a therapeutically effective amount of any of the CD96-modified NK-92 cell lines described herein, *e.g.,* in the preceiding paragraphs, thereby treating the cancer. In some embodiments, the method further comprises administering a therapeutic antibody, e.g., a therapeutic monoclonal antibody. In some embodiments, about 1x10⁸ to about 1x10¹¹ cells per m² of body surface area of the patient are administered to the patient.

In a further aspect, the disclosure further provides a kit for treating cancer, wherein the kit comprises (a) any of the CD96-modified NK-92 cell compositions, or cell lines, as disclosed herein, *e.g.,* in the preceding paragraphs, and (b) instructions for use. In some embodiments, the kit further comprises a physiologically acceptable excipient.

In a further aspect, provided herein is a modified NK-92 that comprises a modification that inhibits expression of CD96 and a modification that inhibits expresson of TIGIT. In some embodiments, the modified NK-92 cell comprises an interfering RNA that targets CD96 and inhibits expression of CD96; and an interfering RNA that targets TIGIT and inhibits expression of TIGIT. In some embodiments, the amount of CD96 expressed by the cell is decreased by at least 50%, at least 60%, at least, 70%, or at least 80%, or greater, compared to a counterpart NK-92 cell that does not have the CD96 modification; and the amount of TIGIT expressed by the cell is decreased by at least 50%, at least 60%, at least, 70%, or at least 80%, or greater, compared to a counterpart NK-92 cell that does not have the TIGIT modification. In some embodiments, the modified NK-92 cell is produced by knocking down or knocking out CD96 expression, and knocking down or knocking out TIGIT expression in the cell. In further embodiments, the modified NK cell expresses at least one Fc receptor, or at least one chimeric antigen receptor (CAR), or both at least one Fc receptor and at least one CAR on the cell surface. In some embodiments, the at least one Fc receptor is CD16 or a CD16 polypeptide having a valine at position 176 (as numbered with reference to the precursor full-length human CD16, including the N-terminal methionine as position 1). In some embodiments the at least one Fc receptor is FcγRIII. In particular embodiments, the at least one Fc receptor comprises a polynucleotide sequence encoding a polypeptide having at least 90% sequence identity to amino acids 19-254 of SEQ ID NO: 13 and comprises a valine at position 176 as numbered with reference to SEQ ID NO: 13. In additional embodiments, the modified NK-92 expresses a CAR that comprises a cytoplasmic domain of FcεRIγ. In some embodiments, the CAR targets a tumor-associated antigen. In further embodiments, the modified NK-92 cell further expresses a cytokine, such as interleukin-2 or a variant thereof. In some embodiments, the cytokine, e.g., IL-2, is targeted to the endoplasmic reticulum.

In a further aspect, the disclosure provides a composition comprising a plurality of modified NK-92 cells as described herein that have reduced CD96 and TIGIT expression as described herein, *e.g.,* in the preceding paragraph. In some embodiments, such a composition comprises a physiologically acceptable excipient.

In another aspect, the disclosure provides a cell line comprising a plurality of modified NK-92 cells as described herein, *e.g.,* in the preceding paragraph, that have reduced CD96 and TIGIT expression. In some embodiments, the cells undergo fewer than 10 population doublings.

In other aspects, the disclosure provides kits, compositions and treatments for cancer with NK-92 cells modified as described herein to reduce CD96 and TIGIT expression. Thus, in some embodiments, provided herein is a composition or cell line for use in a method of treating cancer in a patient in need thereof, the method comprising administering to the patient a therapeutically effective amount of the composition or cell line comprising the NK-92 cells modified to reduce both CD96 and TIGIT expression, thereby treating the cancer. In some embodiments, the method further comprises administering a therapeutic antibody, e.g., a therapeutic monoclonal antibody. In some embodiments, about 1x10⁸ to about 1x10¹¹ cells per m² of body surface area of the patient are administered to the patient.

In a further aspect, the disclosure provides a method for producing an NK-92 cell that expresses decreased levels of CD96 and TIGIT. In some embodiments, the step of genetically modifying the expression of each of the CD96 and TIGIT target genes comprises contacting a NK-92 cell to be modified with an interfering RNA targeting each of target genes. In some embodiments, the interfering RNA targeting each of the one or more target genes is an siRNA, an shRNA, a microRNA, or a single stranded interfering RNA. In particular embodiments, the step of genetically modifying the expression of each of CD96 and TIGIT target genes comprises modifying each of the target genes with a zinc finger nuclease (ZFN), a Tale-effector domain nuclease (TALEN), or a CRIPSR/Cas system. In some embodiments, genetically modifying the expression of each of the target genes comprises: i) introducing a clustered regularly interspaced short palindromic repeat-associated (Cas) protein into the NK-92 cell and ii) introducing one or more ribonucleic acids in the NK-92 cell to be modified, wherein the ribonucleic acids direct the Cas protein to hybridize to a target motif of the sequence of the each of the one or more target genes, and wherein the target motif is cleaved. In some embodiments the Cas protein is introduced into the NK-92 cell in protein form. In other embodiments, the Cas protein is introduced into the NK-92 cell by introducing a Cas coding sequence. Illustrative Cas proteins include Cas9. In some emboidments, the target motif is a 20-nucleotide DNA sequence. The target motif may be, for example, an exon of each of the target genes. In some embodiments, the one or more ribonucleic acids that hybridize to a target motif in CD96 are selected from the group consisting of SEQ ID NOs. 1-4 and the one or more ribonucleic acids that hybridize to a target motif in TIGIT are selected from the group consisting of SEQ ID NOs. 5-8.

The disclosure also provides a CD226-modified NK-92 cell that comprises a modification that inhibits expression of CD226. In some embodiments, the CD226-modified NK-92 cells comprise an interfering RNA that targets CD226 and inhibits its expression. In some embodiments, the amount of CD226 expressed by the CD226-modified NK-92 cell is decreased by at least 20%, at least 30%, at least 50%, at least 60%, or at least 80%, or greater, compared to NK-92 cells that do not have the CD226-targeted alteration. In some embodiments, the CD226-modified NK-92 cell is produced by knocking down or knocking out CD226 in an NK-92 cell. In some embodiments, the step of genetically modifying CD226 expression comprises modifying a CD226 gene with a zinc finger nuclease (ZFN), a Tale-effector domain nuclease (TALEN), or a CRIPSR/Cas system. In some embodiments, genetically modifying CD226 gene expression comprises: i) introducing a clustered regularly interspaced short palindromic repeat-associated (Cas) protein into the NK-92 cell and ii) introducing one or more ribonucleic acids in the NK-92 cell to be modified, wherein the ribonucleic acids direct the Cas protein to hybridize to a target motif of a CD226 gene sequence, and wherein the target motif is cleaved. In some embodiments, the Cas protein is introduced into the NK-92 cell in protein form. In some embodiments, the Cas protein is introduced into the NK-92 cell by introducing a Cas nucleic acid coding sequence. In some embodiments, the Cas protein is Cas9. In some embodiments, the target motif is a 20 nucleotide DNA sequence

The foregoing general description and the following detailed description are exemplary and explanatory and are intended to provide further explanation of the invention as claimed. Other objects, advantages and novel features will be readily apparent to those skilled in the art from the following detailed description of the invention.

### BRIEF DESCRIPTION OF THE DRAWINGS

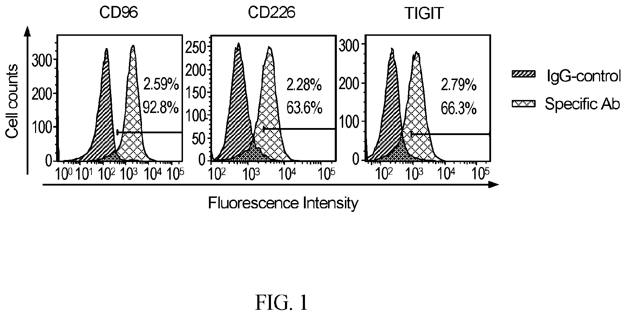

Figure 1 provides data showing NK-92 cells express the activating CD226, and inhibitory CD96, and TIGIT receptors. Flow cytometry analysis of CD226, CD96 and TIGIT expression in NK-92 cells was performed as described in "Materials and Methods".

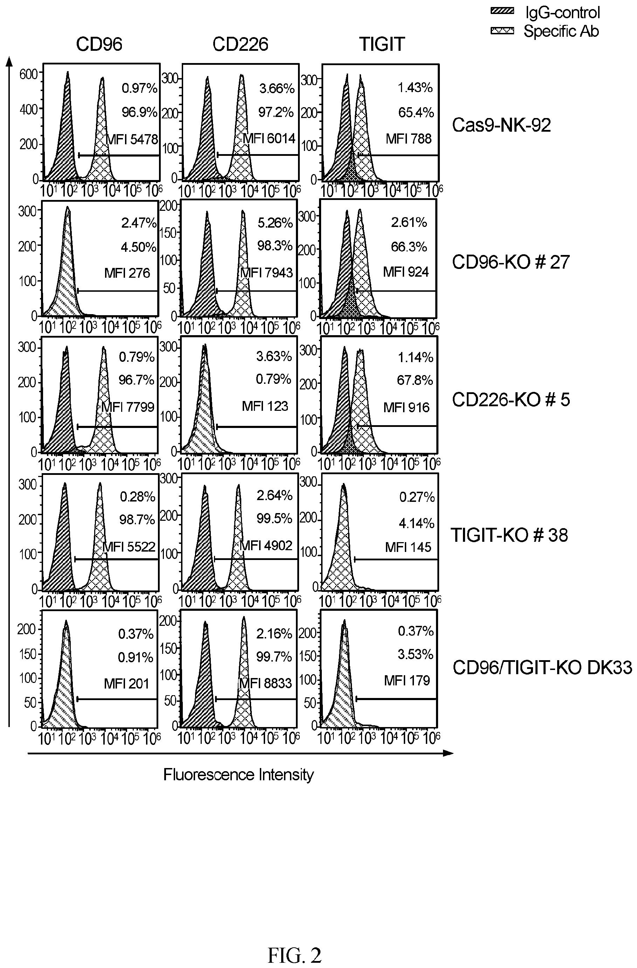

Figure 2 provides data illustrating CD226, CD96, and TIGIT receptor expression in parental and knock-out NK-92 cells. Flow cytometry analysis of CD226, CD96 and TIGIT expression in parental Cas9-NK-92 or nectin receptor knock-out NK-92 cells was performed as described in "Materials and Methods". The MFI (Mean Fluorescence Intensity) values for the samples stained with the specific antibody are also indicated.

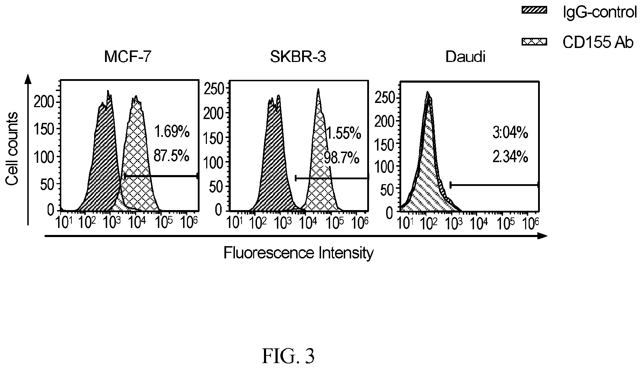

Figure 3 provides data showing CD155 expression in MCF-7, SKBR-3, and Daudi tumor cells. Flow cytometry analysis of CD155 expression in MCF-7, SKBR-3, and Daudi cells was performed as described in "Materials and Methods".

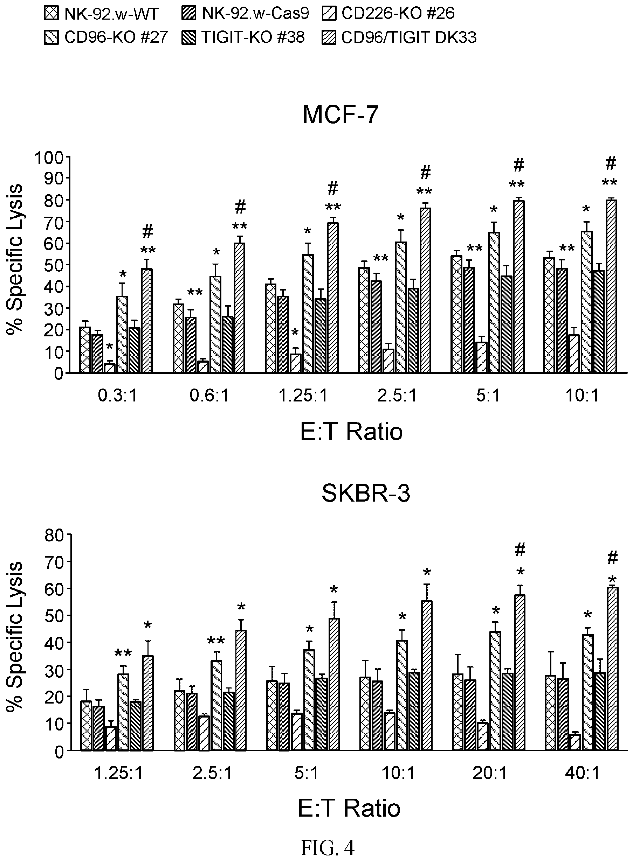

Figure 4 provide data illustrating that CD96 and CD96/TIGIT KO NK-92 cells have a higher cytotoxic potential against CD155-positive tumor targets. The ability of parental or nectin receptor KO NK-92 cells to kill CD155-positive MCF-7 (top) or SKBR-3 (bottom) tumor cells was evaluated in a 4 hour cytotoxicity assay at different effector to target (E:T) ratios. Each set of bars represents the following cell types, listed from left to right: NK-92-WT, NK-92-Cas9, CD226-KO, CD96-KO, TIGIT-KO, CD96/TIGIT double knockout. The bar graphs show the average +/- SEM of three independent experiments. P-values were calculated using the Student's t-test. (*), indicates p<0.05; (**), indicates p<0.01, both relative to parental NK-92 cells. (#), indicates p<0.05 relative to CD96-KO cells.

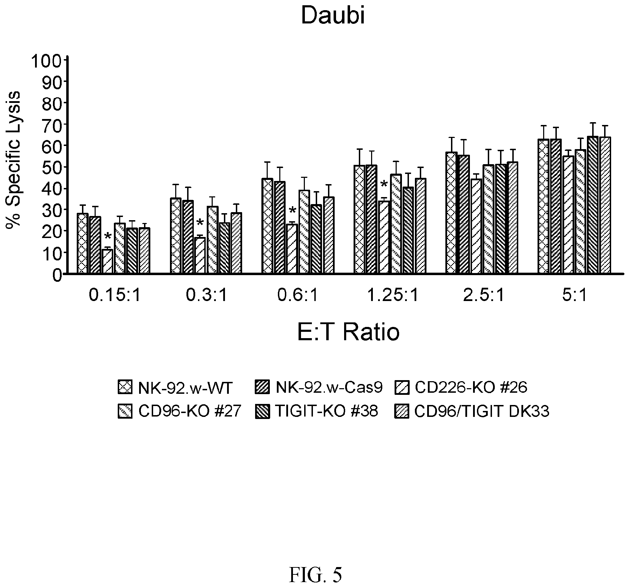

Figure 5 provides data showing that the increased cytotoxic potential of CD96 and CD96/TIGIT KO NK-92 cells is specific to the loss of the nectin binding receptors. The ability of parental or nectin receptor KO NK-92 cells to kill CD155-negative Daudi tumor cells was evaluated in a 4 hour cytotoxicity assay at different effector to target (E:T) ratios. Each set of bars represents the following cell types, listed from left to right: NK-92-WT, NK-92-Cas9, CD226-KO, CD96-KO, TIGIT-KO, CD96/TIGIT double knockout. The bar graph shows the average +/- SEM of four independent experiments. P-values were calculated using the Student's t-test. (*), indicates p<0.05 relative to parental NK-92 cells.

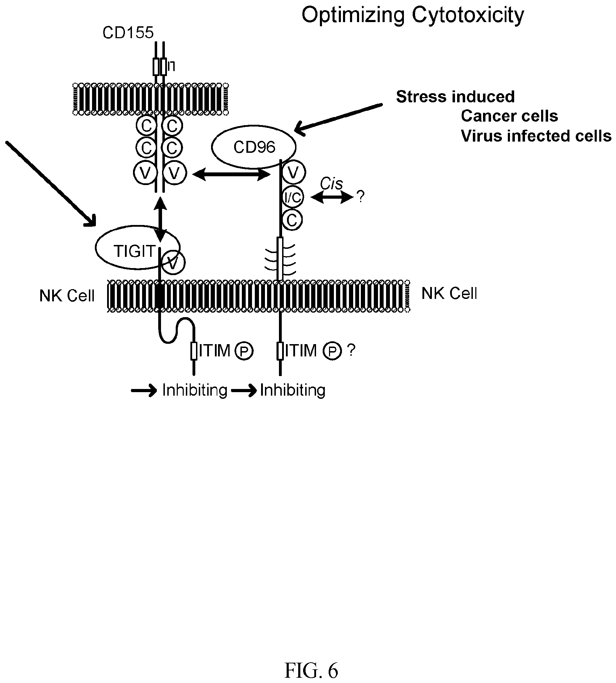

Figure 6 is an schematic illustration of the roles of TIGIT and CD96 in NK cells function.

### DETAILED DESCRIPTION OF THE INVENTION

In one aspect, the present invention provides CD96-modified NK-92 cells having decreased CD96 expression and optionally also s having decreased TIGIT expression, and methods of producing such cells. CD96-modified NK-92 cells in accordance with the present disclosure have a CD96-targeted alteration in the NK-92 cells; and additionally TIGIT-modified NK-92 cells further have a TIGIT-targeted alteration in the NK-92 cells. The invention further provides method of using the modified NK-92 cells for the treatment of cancer.

### TERMINOLOGY

Unless defined otherwise, all technical and scientific terms used herein have the same meaning as commonly understood by one of ordinary skill in the art to which this invention belongs.

In this specification and in the claims that follow, reference will be made to a number of terms that shall be defined to have the following meanings:

The terminology used herein is for the purpose of describing particular embodiments only and is not intended to be limiting of the invention. As used herein, the singular forms "a", "an" and "the" are intended to include the plural forms as well, unless the context clearly indicates otherwise.

All numerical designations, e.g., pH, temperature, time, concentration, amounts, an molecular weight, including ranges, are approximations which are varied (+) or (-) by increments of 0.1 or 1.0, where appropriate. It is to be understood, although not always explicitly stated, that all numerical designations may be preceded by the term "about." It is also to be understood, although not always explicitly stated, that the reagents described herein are merely exemplary and that equivalents of such are known in the art.

"Optional" or "optionally" means that the subsequently described event or circumstance can or cannot occur, and that the description includes instances where the event or circumstance occurs and instances where it does not.

The term "comprising" is intended to mean that the compositions and methods include the recited elements, but do not exclude others. "Consisting essentially of" when used to define compositions and methods, refers to the specified materials or steps and those that do not materially affect the basic and novel characteristic(s) of the claimed invention. "Consisting of" shall mean excluding more than trace amounts of other ingredients and substantial method steps recited. Embodiments defined by each of these transition terms are within the scope of this invention.

The term "natural killer (NK) cells" refers to cells of the immune system that kill target cells in the absence of a specific antigenic stimulus, and without restriction according to MHC class. Target cells may be tumor cells or cells harboring viruses. NK cells are characterized by the presence of CD56 and the absence of CD3 surface markers.

The term "NK-92 cells" refers to the NK cell line, NK-92, which was originally obtained from a patient having non-Hodgkin's lymphoma. For purposes of this invention and unless indicated otherwise, the term "NK-92" is intended to refer to the original NK-92 cell lines as well as NK-92 cell lines, clones of NK-92 cells, and NK-92 cells that have been modified (*e.g.,* by introduction of exogenous genes). NK-92 cells and exemplary and non-limiting modifications thereof are described in U.S. Patent Nos. 7,618,817; 8,034,332; and 8,313,943.

As used herein, the term "CD96-targeted alteration" refers to a change to the structure or properties of DNA or RNA of CD96 in a NK-92 cell, for example, knocking out or knocking down CD96 expression, which leads to a decrease in the level of CD96 protein. Thus, a CD96-targeted alteration can target a CD96 gene or a CD96 gene transcript. An example of a human CD96 protein sequence is available under Uniprotein number P40200. Human CD96 is located on chromosome 3 and is mapped to region 3q13.13-q13.2 by HGNC. CD96 is at location Chr 3 NC_000003.12 (111542079..111665996) according to the Genome Reference Consortium Human Build 38 patch release 7 (GRCh38.p7) assembly. The term "CD96" also encompasses allelic variants, including transcript variants 2 and 3, encoded by the CD96 gene.

The term "CD96-modified NK-92 cell" refers to an NK-92 cell that has a CD96-targeted alteration that results in a decrease in amount of CD96 expression. The genetically modified NK-92 cells may further other genetic alterations, e.g., a modification that decreases TIGIT expression, or a transgene encoding a suicide gene, and Fc receptor, or chimeric antigen receptor (CAR).

The term "CD96-unmodified NK-92 cells" refers to the NK-92 cells that do not have a CD96-targeted alteration.

The term "TIGIT-targeted alteration" as used herein refers to a change to the structure or properties of DNA or RNA of TIGIT in a NK-92 cell, for example, knocking out or knocking down TIGIT expression, which leads to a decrease in the level of TIGIT protein. Thus, a TIGIT-targeted alteration can target a TIGIT gene or a TIGIT gene transcript. An example of a human TIGIT protein sequence is available under Uniprotein number Q495A1. Human TIGIT is located on chromosome 3 and is mapped to region 3q13.31. TIGIT is at chr3 NC_000003.12 (114293986..114310288) according to the Genome Reference Consortium Human Build 38 patch release 7 (GRCh38.p7) assembly. The term "TIGIT" also encompasses allelic variants of the exemplary references sequence that are encoded by a gene at the TIGIT chromosomal locus.

The term "TIGIT-modified NK-92 cell" refers to an NK-92 cell that has a TIGIT-targeted alteration that results in a decrease in amount of TIGIT expression. The genetically modified NK-92 cells may further other genetic alterations, e.g., a modification that decreases CD96 expression, or a transgene encoding an Fc receptor, a suicide gene or chimeric antigen receptor (CAR).

The term "TIGIT-unmodified NK-92 cells" refers to the NK-92 cells that do not have a TIGIT-targeted alteration.

The term "non-irradiated NK-92 cells" refers to NK-92 cells that have not been irradiated. Irradiation renders the cells incapable of growth and proliferation. In some embodiments, it is envisioned that the NK-92 cells for administration will be irradiated at a treatment facility or some other point prior to treatment of a patient, since the time between irradiation and infusion should be no longer than four hours in order to preserve optimal activity. Alternatively, NK-92 cells may be inactivated by another mechanism.

As used herein, "inactivation" of the NK-92 cells renders them incapable of growth. Inactivation may also relate to the death of the NK-92 cells. It is envisioned that the NK-92 cells may be inactivated after they have effectively purged an *ex vivo* sample of cells related to a pathology in a therapeutic application, or after they have resided within the body of a mammal a sufficient period of time to effectively kill many or all target cells residing within the body. Inactivation may be induced, by way of non-limiting example, by administering an inactivating agent to which the NK-92 cells are sensitive.

As used herein, the terms "cytotoxic" and "cytolytic", when used to describe the activity of effector cells such as NK cells, are intended to be synonymous. In general, cytotoxic activity relates to killing of target cells by any of a variety of biological, biochemical, or biophysical mechanisms. Cytolysis refers more specifically to activity in which the effector lyses the plasma membrane of the target cell, thereby destroying its physical integrity. This results in the killing of the target cell. Without wishing to be bound by theory, it is believed that the cytotoxic effect of NK cells is due to cytolysis.

The term "kill" with respect to a cell/cell population is directed to include any type of manipulation that will lead to the death of that cell/cell population.

The term "Fc receptor" refers to a protein found on the surface of certain cells (e.g., natural killer cells) that contribute to the protective functions of the immune cells by binding to part of an antibody known as the Fc region. Binding of the Fc region of an antibody to the Fc receptor (FcR) of a cell stimulates phagocytic or cytotoxic activity of a cell via antibody-mediated phagocytosis or antibody-dependent cell-mediated cytotoxicity (ADCC). FcRs are classified based on the type of antibody they recognize. For example, Fc-gamma receptors (FCγR) bind to the IgG class of antibodies. FCγRIII-A (also called CD16) is a low affinity Fc receptor bind to IgG antibodies and activate ADCC. FCγRIII-A are typically found on NK cells.

The terms "polynucleotide", "nucleic acid" and "oligonucleotide" are used interchangeably and refer to a polymeric form of nucleotides of any length, either deoxyribonucleotides or ribonucleotides or analogs thereof. Polynucleotides can have any three dimensional structure and may perform any function, known or unknown. The following are non limiting examples of polynucleotides: a gene or gene fragment (for example, a probe, primer, EST or SAGE tag), exons, introns, messenger RNA (mRNA), transfer RNA, ribosomal RNA, ribozymes, cDNA, recombinant polynucleotides, branched polynucleotides, plasmids, vectors, isolated DNA of any sequence, isolated RNA of any sequence, nucleic acid probes and primers. A polynucleotide can comprise modified nucleotides, such as methylated nucleotides and nucleotide analogs. If present, modifications to the nucleotide structure can be imparted before or after assembly of the polynucleotide. The sequence of nucleotides can be interrupted by non nucleotide components. A polynucleotide can be further modified after polymerization, such as by conjugation with a labeling component. The term also refers to both double and single stranded molecules. Unless otherwise specified or required, any embodiment of this invention that is a polynucleotide encompasses both the double stranded form and each of two complementary single stranded forms known or predicted to make up the double stranded form.

A polynucleotide is composed of a specific sequence of four nucleotide bases: adenine (A); cytosine (C); guanine (G); thymine (T); and uracil (U) for thymine when the polynucleotide is RNA. Thus, the term "polynucleotide sequence" is the alphabetical representation of a polynucleotide molecule.

The term "percent identity" refers to sequence identity between two peptides or between two nucleic acid molecules. Percent identity can be determined by comparing a position in each sequence which may be aligned for purposes of comparison. When a position in the compared sequence is occupied by the same base or amino acid, then the molecules are identical at that position. As used herein, the phrase "homologous" or "variant" nucleotide sequence," or "homologous" or "variant" amino acid sequence refers to sequences characterized by identity, at the nucleotide level or amino acid level, of at least a specified percentage. Homologous nucleotide sequences include those sequences coding for naturally occurring allelic variants and mutations of the nucleotide sequences set forth herein. Homologous nucleotide sequences include nucleotide sequences encoding for a protein of a mammalian species other than humans. Homologous amino acid sequences include those amino acid sequences which contain conservative amino acid substitutions and which polypeptides have the same binding and/or activity. In some embodiments, a homologous nucleotide or amino acid sequence has at least 60% or greater, for example at least 70%, or at least 80%, at least 85% or greater, with a comparator sequence. In some embodiments, a homologous nucleotide or amino aicd sequence has at leaset 90%, 91%, 92%, 93%, 94%, 95%, 96%, 97%, 98% or 99% dentity with a comparator sequence. In some embodiments, a homologous amino acid sequence has no more than 15, nor more than 10, nor more than 5 or no more than 3 conservative amino acid substitutions. Percent identity can be determined by, for example, the Gap program (Wisconsin Sequence Analysis Package, Version 8 for UNIX, Genetics Computer Group, University Research Park, Madison Wis.), using default settings, which uses the algorithm of Smith and Waterman (Adv. Appl. Math., 1981, 2, 482-489).

The term "express" refers to the production of a gene product such as RNA or protein. The term "transient expression" refers to expression from a polynucleotide is not incorporated into the genome of the cell.

The term "cytokine" or "cytokines" refers to the general class of biological molecules which effect cells of the immune system. Exemplary cytokines for use in practicing the invention include but are not limited to interferons and interleukins (IL), in particular IL-2, IL-12, IL-15, IL-18 and IL-21.

The term "vector" refers to a non-chromosomal nucleic acid comprising an intact replicon such that the vector may be replicated when placed within a permissive cell, for example by a process of transformation. A vector may replicate in one cell type, such as bacteria, but have limited ability to replicate in another cell, such as mammalian cells. Vectors may be viral or non-viral. Exemplary non-viral vectors for delivering nucleic acid include naked DNA; DNA complexed with cationic lipids, alone or in combination with cationic polymers; anionic and cationic liposomes; DNA-protein complexes and particles comprising DNA condensed with cationic polymers such as heterogeneous polylysine, defined-length oligopeptides, and polyethylene imine, in some cases contained in liposomes; and the use of ternary complexes comprising a virus and polylysine-DNA.

The term "target motif" refers to a nucleic acid sequence that defines a portion of a nucleic acid to which a binding molecule will bind, provided sufficient conditions for binding exist.

The term "interfering RNA" refers to an RNA nucleic acid molecule which is double stranded or single stranded and is capable of effecting the induction of an RNA interference mechanism directed to knocking down the expression of a target gene.

The terms "patient," "subject," "individual," and the like are used interchangeably herein, and refer to any animal, or cells thereof whether in vitro or in situ, amenable to the methods described herein. In certain non-limiting embodiments, the patient, subject or individual is a human.

The term "recipient," refers a patient who is administered NK-92 cells,whether modified or unmodified, during treatment.

The term "treating" or "treatment" covers the treatment of a disease or disorder described herein, in a subject, such as a human, and includes: (i) inhibiting a disease or disorder, i.e., arresting its development; (ii) relieving a disease or disorder, i.e., causing regression of the disorder; (iii) slowing progression of the disorder; and/or (iv) inhibiting, relieving, or slowing progression of one or more symptoms of the disease or disorder.

The term "administering" or "administration" of a therapeutic agent such as NK-92 cells, includes any route of introducing or delivering the therapeutic agent to perform the intended function. Administration can be carried out by any route suitable for the delivery of the agent. Thus, delivery routes can include intravenous, intramuscular, intraperitoneal, or subcutaneous deliver. In some embodiments NK-92 cells are administered directly to the tumor, *e.g.,* by injection into the tumor.

The term "contacting" (i.e., contacting a polynucleotide sequence with a clustered regularly interspaced short palindromic repeats-associated (Cas) protein and/or ribonucleic acids) is intended to include incubating the Cas protein and/or the ribonucleic acids in the cell together in vitro (e.g., adding the Cas protein or nucleic acid encoding the Cas protein to cells in culture). In some embodiments, the term "contacting" is not intended to include the in vivo exposure of cells to the Cas protein and/or ribonucleic acids as disclosed herein that may occur naturally in a microorganism (i.e., bacteria). The step of contacting a target polynucleotide sequence with a Cas protein and/or ribonucleic acids as disclosed herein can be conducted in any suitable manner. For example, the cells may be treated in adherent culture, or in suspension culture. It is understood that the cells contacted with a Cas protein and/or ribonucleic acids as disclosed herein can also be simultaneously or subsequently contacted with another agent, such as a growth factor or other differentiation agent or environments to stabilize the cells, or to differentiate the cells further.

The term "knock out" as used herein includes deleting all or a portion of the target polynucleotide sequence in a way that interferes with the function of the target polynucleotide sequence. For example, a knock out can be achieved by altering a target polynucleotide sequence by inducing a deletion in the target polynucleotide sequence in a functional domain of the target polynucleotide sequence. Those skilled in the art will readily appreciate how to use various genetic approaches, e.g., CRISPR/Cas systems, ZFN, TALEN, TgAgo, to knock out a target polynucleotide sequence or a portion thereof based upon the details described herein.

The term "knock down" as used herein refers to a measurable reduction in expression of a target mRNA or the corresponding protein in a genetically modified cell as compared with the expression of the target mRNA or the corresponding protein in a counterpart control cell that does not contain the genetic modification to reduce expression. Those skilled in the art will readily appreciate how to use various genetic approaches, e.g., siRNA, shRNA, microRNA, antisense RNA, or other RNA-mediated inhibition techniques, to knock down a target polynucleotide sequence or a portion thereof based upon the details described herein.

The terms "decrease," "reduced," "reduction," and "decrease" are all used herein to refer to a decrease by at least 10% as compared to a reference level, for example a decrease by at least about 20%, or at least about 30%, or at least about 40%, or at least about 50%, or at least about 60%, or at least about 70%, or at least about 80%, or at least about 90% or up to and including a 100% decrease (i.e. absent level as compared to a reference sample), or any decrease between 10-100% as compared to a reference level.

The term "cancer" refers to all types of cancer, neoplasm, or malignant tumors found in mammals, including leukemia, lymphomas, carcinomas, and sarcomas. Exemplary cancers include cancer of the brain, breast, cervix, colon, head & neck, liver, kidney, lung, non-small cell lung, melanoma, mesothelioma, ovary, sarcoma, stomach, uterus and Medulloblastoma. Additional examples include, Hodgkin's Disease, Non-Hodgkin's Lymphoma, multiple myeloma, neuroblastoma, ovarian cancer, rhabdomyosarcoma, primary thrombocytosis, primary macroglobulinemia, primary brain tumors, cancer, malignant pancreatic insulanoma, malignant carcinoid, urinary bladder cancer, premalignant skin lesions, testicular cancer, lymphomas, thyroid cancer, neuroblastoma, esophageal cancer, genitourinary tract cancer, malignant hypercalcemia, endometrial cancer, adrenal cortical cancer, neoplasms of the endocrine and exocrine pancreas, and prostate cancer.

### NK-92 CELLS

The NK-92 cell line is a human, IL-2-dependent NK cell line that was established from the peripheral blood mononuclear cells (PBMCs) of a 50-year-old male diagnosed with non-Hodgkin lymphoma (Gong, et al., Leukemia. 8:652-8 (1994)). The NK-92 cell line expresses CD56^{bright}, CD2, CD7, CD11a, CD28, CD45, and CD54 surface markers, but does not display CD1, CD3, CD4, CD5, CD8, CD10, CD14, CD16, CD19, CD20, CD23, and CD34 markers. Unlike normal NK cells, NK-92 lacks expression of most killer cell inhibitor receptors (KIRs) (Maki, et al., J Hematother Stem Cell Res. 10:369-83 (2001)). Only KIR2DL4, a KIR receptor with activating function and inhibitory potential that is expressed by all NK cells, was detected on the surface of NK-92 cells.

Growth of NK-92 cells in culture is dependent upon the presence of recombinant interleukin 2 (rIL-2), with a dose as low as 1 IU/mL being sufficient to maintain proliferation. IL-7 and IL-12 do not support long-term growth, nor do other cytokines tested, including IL-1α, IL-6, tumor necrosis factor α, interferon α, and interferon γ. NK-92 has high cytotoxicity even at a low effector:target (E:T) ratio of 1:1. Gong, *et al., supra.*

NK-92 cells include, but are not limited to, those described in, e.g., U.S. Patent Nos. 7,618,817, 8,034,332, and 8,313,943, US Patent Application Publication No. 2013/0040386. NK92 cells are known and readily available to a person of ordinary skill in the art from NantKwest, Inc. Illustrative NK-92 cell lines include wild type NK-92, NK-92-CD16, NK-92-CD16-γ, NK-92-CD16-ζ, NK-92-CD16(F176V), NK-92MI and NK-92CI.

### DECREASING EXPRESSION OF CD96

The instant disclosure provides a CD96-modified NK-92 cell comprising a CD96-targeted alteration that inhibits expression of CD96. In some embodiments, the CD96-modified NK-92 cell is generated by disruption of a CD96 gene. Methods for disrupting a CD96 gene include, but are not limited to, methods employing a zinc finger nuclease (ZFN), a Tale-effector domain nuclease (TALEN), and CRIPSR/Cas system.

### CRISPR

In some embodiments, the knocking out or knocking down of CD96 is peformed using CRISPR/CAS methodology. A CRISPR/Cas system includes a Cas protein and at least one to two ribonucleic acids that are capable of directing the Cas protein to and hybridizing to a target motif in the CD96 gene sequence. The Cas protein then cleaves the target motif and result in a double-strand break or a single-strand break results. Any CRISPR/Cas system that is capable of altering a target polynucleotide sequence in a cell can be used. In some embodiments, the CRISPR Cas system is a CRISPR type I system, in some embodiments, the CRISPR/Ca system is a CRISPR type II system. In some embodiments, the CRISPR/Cas system is a CRISPR type V system.

The Cas protein used in the invention can be a naturally occurring Cas protein or a functional derivative thereof. A "functional derivative" includes, but are not limited to, fragments of a native sequence and derivatives of a native sequence polypeptide and its fragments, provided that they have a biological activity in common with a corresponding native sequence polypeptide. A biological activity contemplated herein is the ability of the functional derivative to hydrolyze a DNA substrate into fragments. The term "derivative" encompasses both amino acid sequence variants of polypeptide, covalent modifications, and fusions thereof such as derivative Cas proteins. Suitable derivatives of a Cas polypeptide or a fragment thereof include but are not limited to mutants, fusions, covalent modifications of Cas protein or a fragment thereof.

In some embodiments, the Cas protein used in the invention is Cas9 or a functional derivative thereof. In some embodiments, the Cas9 protein is from *Streptococcus pyogenes.* Cas 9 contains 2 endonuclease domains, including an RuvC-like domain which cleaves target DNA that is noncomplementary to crRNA, and an HNH nuclease domain which cleave target DNA complementary to crRNA. The double-stranded endonuclease activity of Cas9 also requires that a short conserved sequence, (2-5 nucleotides), known as a protospacer-associated motif (PAM), follows immediately 3'- of a target motif in the target sequence.

In some embodiments, the Cas protein is introduced into the NK-92 cells in polypeptide form. In certain embodiments, the Cas proteins can be conjugated to or fused to a cell-penetrating polypeptide or cell-penetrating peptide that is well known in the art. Non-limiting examples of cell-penetrating peptides include those provided in Milletti F, "Cell-penetrating peptides: classes, orgin and current landscape." Drug Discov. Today 17: 850-860, 2012. In some cases, an unmodified NK-92 cell is genetically engineered to produce the Cas protein.

In some embodiments, the target motif in the target gene, to which the Cas protein is directed by the guide RNAs, is 17 to 23 bp in length. In some embodiments, the target motif is at least 20 bp in length. In some embodiments, the target motif is a 20-nucleotide DNA sequence. In some embodiments, the target motif is a 20-nucleotide DNA sequence and immediately precedes a short conserved sequence known as a protospacer-associated motif (PAM), recognized by the Cas protein. In some embodiments, the PAM motif is an NGG motif. In some embodiments, the target motif of the target gene is within an exon.

In some embodiments, the target motifs can be selected to minimize off-target effects of the CRISPR/Cas systems of the present invention. In some embodiments, the target motif is selected such that it contains at least two mismatches when compared with all other genomic nucleotide sequences in the cell. In some embodiments, the target motif is selected such that it contains at least one mismatch when compared with all other genomic nucleotide sequences in the cell. Those skilled in the art will appreciate that a variety of techniques can be used to select suitable target motifs for minimizing off-target effects (e.g., bioinformatics analyses).

The ribonucleic acids that are capable of directing the Cas protein to and hybridizing to a target motif in the target gene sequence are referred to as single guide RNA ("sgRNA"). The sgRNAs can be selected depending on the particular CRISPR/Cas system employed, and the sequence of the target polynucleotide, as will be appreciated by those skilled in the art. In some embodiments, the one to two ribonucleic acids can also be selected to minimize hybridization with nucleic acid sequences other than the target polynucleotide sequence. In some embodiments, the one to two ribonucleic acids hybridize to a target motif that contains at least two mismatches when compared with all other genomic nucleotide sequences in the cell. In some embodiments, the one to two ribonucleic acids hybridize to a target motif that contains at least one mismatch when compared with all other genomic nucleotide sequences in the cell. In some embodiments, the one to two ribonucleic acids are designed to hybridize to a target motif immediately adjacent to a deoxyribonucleic acid motif recognized by the Cas protein. In some embodiments, each of the one to two ribonucleic acids are designed to hybridize to target motifs immediately adjacent to deoxyribonucleic acid motifs recognized by the Cas protein which flank a mutant allele located between the target motifs. Guide RNAs can also be designed using software that are readily available, for example, at the website crispr.mit.edu. The one or more sgRNAs can be transfected into the NK-92 cells in which Cas protein is present by transfection, according to methods known in the art. In some embodiments, the sgRNAs that target CD96 are one or more sgRNAs selected from the group consisting of SEQ ID NOs: 1-4.

Methods of using the CRISPR/Cas system to reduce gene expression are described in various publications, e.g., U.S. Patent Application Publication No. 2014/0170753.

### Zinc finger nuclease (ZFN)

**In** some embodiments, the modified NK-92 cells comprising a CD96-targeted alteration are produced by knocking out CD96 in NK-92 cells using a zinc finger nuclease (ZFN). ZFNs are fusion proteins that comprise a non-specific cleavage domain (N) of FokI endonuclease and a zinc finger protein (ZFP). A pair of ZNFs are involved to recognize a specific locus in a target gene -- one that recognizes the sequence upstream and the other that recognizes the sequence downstream of the site to be modified-and the nuclease portion of the ZFN cuts at the specific locus and causing the knock out of the target CD96 gene. Methods of using the ZFNs to reduce gene expression is well known, for example, as disclosed in U.S. Patent No. 9,045,763, and also in Durai et al., "Zinc Finger Nucleases: Custom-Designed Molecular Scissors for Genome Engineering of Plant and Mamalian cells," Nucleic Acid Research 33 (18):5978-5990 (2005).

### Transcription activator-like effector nucleases (TALENS)

In some embodiments, CD96-modified NK-92 cells comprising a targeted alteration are produced by knocking out CD96 with transcription activator-like effector nucleases (TALENS). TALENs are similar to ZFNs in that they bind as a pair around a genomic site and direct the same non-specific nuclease, FoKI, to cleave the genome at a specific site, but instead of recognizing DNA triplets, each domain recognizes a single nucleotide. Methods of using the ZFNs to reduce gene expression are also well known, for example, as disclosed in U.S. Patent No. 9,005,973, and also Christian et al. "Targeting DNA Double-Strand Breaks with TAL Effector Nulceases," Genetics 186(2): 757-761 (2010).

### Knocking down CD96 expression in NK-92 cells

In some embodiments, CD96-modified NK-92 cells comprising a targeted alteration are produced by knocking down one CD96 with an interfering RNA. Interfering RNAs, when introduced *in vivo,* form an RNA-inducing silencing complex ("RISC") with other proteins and initiate a process known as RNA interference (RNAi). During the RNAi process, the RISC incorporates a single-stranded interfering RNA or one strand of a double stranded interfering RNA. The incorporated strand acts as a template for RISC to recognize complementary mRNA transcript. Once the complementary mRNA is identified, the protein components in RISC activate and cleave the mRNA, resulting in a knock-down of target gene expression. Non-limiting examples of interfering RNA molecules that be used to knock down expression of the target gene include siRNAs, short hairpin RNAs (shRNAs), single stranded interfering RNAs, and microRNAs (miRNAs). Methods for using these interfering RNAs are well known to one of skilled in the art.

In one embodiment, the interfering RNA is a siRNA. siRNA is a double stranded RNA which is typically less than 30 nucleotides long. Gene silencing by siRNA starts with one strand of the siRNA being incorporated into a ribonucleoprotein complex known as the RNA-induced silencing complex (RISC). The strand incorporated in RISC identifies mRNA molecules that are at least partially complementary to the incorporated siRNA strand and the RISC then cleaves these target mRNAs or inhibits their translation.

In one embodiment, the interfering RNA is a microRNA. microRNA is a small noncoding RNA molecule, which can hybridize to complementary sequences within mRNA molecules, resulting cleavage of the mRNA, or destabilization of the mRNA through shortening of its poly(A) tail.

In one embodiment, the interfering RNA is a single-stranded interfering RNA. The single strand can also effect mRNA silencing in a manner that is similar to the double stranded siRNA, albeit less efficient than, the double-stranded siRNA. The single-stranded interfering RNA typically has a length of about 19 to about 49 nucleotides as for the double-stranded siRNA described above.

A short hairpin RNA or small hairpin RNA (shRNA) is an artificial RNA molecule with a tight hairpin turn that can be used to silence target gene expression via the siRNA it produced in cells. Expression of shRNA in cells is typically accomplished by delivery of plasmids or through viral or bacterial vectors. Suitable vectors include but not limited to adeno-associated viruses (AAVs), adenoviruses, and lentiviruses. shRNA is an advantageous mediator of siRNA in that it has relatively low rate of degradation and turnover.

Interfering RNAs used herein may differ from naturally-occurring RNA by the addition, deletion, substitution or modification of one or more nucleotides. Non-nucleotide material may be bound to the interfering RNA, either at the 5' end, the 3' end, or internally. Non-limiting examples of modifications that interfering RNAs may contain relative to the naturally -occurring RNA are disclosed in U.S. Patent No. 8,399,653. Such modifications are commonly designed to increase the nuclease resistance of the interfering RNAs, to improve cellular uptake, to enhance cellular targeting, to assist in tracing the interfering RNA, to further improve stability, or to reduce the potential for activation of the interferon pathway. For example, interfering RNAs may comprise a purine nucleotide at the ends of overhangs. Conjugation of cholesterol to the 3' end of the sense strand of an siRNA molecule by means of a pyrrolidine linker, for example, also provides stability to an siRNA.

Interfering RNAs used herein are typically about 10-60, 10-50, or 10-40 (duplex) nucleotides in length, more typically about 8-15, 10-30, 10-25, or 10-25 (duplex) nucleotides in length, about 10-24, (duplex) nucleotides in length (e.g., each complementary sequence of the double-stranded siRNA is 10-60, 10-50, 10-40, 10-30, 10-25, or 10-25 nucleotides in length, about 10-24, 11-22, or 11-23 nucleotides in length, and the double-stranded siRNA is about 10-60, 10-50, 10-40, 10-30, 10-25, or 10-25 base pairs in length).

Techniques for selecting target motifs in a gene of interest for RNAi are known to those skilled in the art, for example, as disclosed in Tuschl, T. et al., "The siRNA User Guide," revised May 6, 2004, available on the Rockefeller University web site; by Technical Bulletin #506, "siRNA Design Guidelines," Ambion Inc. at Ambion's web site; and by other web-based design tools at, for example, the Invitrogen, Dharmacon, Integrated DNA Technologies, Genscript, or Proligo web sites. Initial search parameters can include G/C contents between 35% and 55% and siRNA lengths between 19 and 27 nucleotides. The target sequence may be located in the coding region or in the 5' or 3' untranslated regions of the mRNA. The target sequences can be used to derive interfering RNA molecules, such as those described herein.

Efficiency of the knock-out or knock-down can be assessed by measuring the amount of CD96 mRNA or protein using methods well known in the art, for example, quantitative PCR, western blot, flow cytometry, etc and the like. In some embodiments, the level of CD96 protein is evaluated to assess knock-out or knock-down efficiency. In certain embodiments, the efficiency of reduction of target gene expression is at least 5%, at least 10%, at least 20% , at least 30%, at least 50%, at least 60%, or at least 80%, or at least 90%, or greater. as compared to corresponding NK-92 cells that do not have the CD96-targeted alteration. In certain embodiments, the efficiency of reduction is from about 10% to about 90%. In certain embodiments, the efficiency of reduction is from about 30% to about 80%. In certain embodiments, the efficiency of reduction is from about 50% to about 80%. In some embodiments, the efficiency of reduction is greater than or equal to about 80%.

### DECREASING EXPRESSION OF TIGIT

The instant disclosure additionally provides, in addition to the CD96 modification, a TIGIT-modified NK-92 cell further comprising a TIGIT-targeted alteration that inhibits expression of TIGIT. In some embodiments, the TIGIT-modified NK-92 cell is generated by disruption of a TIGIT gene. Methods for disrupting a TIGIT gene include, but are not limited to, methods employing a zinc finger nuclease (ZFN), a Tale-effector domain nuclease (TALEN), and CRIPSR/Cas system.

### CRISPR

In some embodiments, the knocking out or knocking down of TIGIT is peformed using CRISPR/CAS methodology. A CRISPR/Cas system includes a Cas protein and at least one to two ribonucleic acids that are capable of directing the Cas protein to and hybridizing to a target motif in the TIGIT gene sequence. The Cas protein then cleaves the target motif and result in a double-strand break or a single-strand break results. Any CRISPR/Cas system that is capable of altering a target polynucleotide sequence in a cell can be used. In some embodiments, the CRISPR Cas system is a CRISPR type I system, in some embodiments, the CRISPR/Ca system is a CRISPR type II system. In some embodiments, the CRISPR/Cas system is a CRISPR type V system.

The Cas protein used in the invention can be a naturally occurring Cas protein or a functional derivative thereof. A "functional derivative" includes, but are not limited to, fragments of a native sequence and derivatives of a native sequence polypeptide and its fragments, provided that they have a biological activity in common with a corresponding native sequence polypeptide. A biological activity contemplated herein is the ability of the functional derivative to hydrolyze a DNA substrate into fragments. The term "derivative" encompasses both amino acid sequence variants of polypeptide, covalent modifications, and fusions thereof such as derivative Cas proteins. Suitable derivatives of a Cas polypeptide or a fragment thereof include but are not limited to mutants, fusions, covalent modifications of Cas protein or a fragment thereof.

In some embodiments, the Cas protein used in the invention is Cas9 or a functional derivative thereof. In some embodiments, the Cas9 protein is from *Streptococcus pyogenes.* Cas 9 contains 2 endonuclease domains, including an RuvC-like domain which cleaves target DNA that is noncomplementary to crRNA, and an HNH nuclease domain which cleave target DNA complementary to crRNA. The double-stranded endonuclease activity of Cas9 also requires that a short conserved sequence, (2-5 nucleotides), known as a protospacer-associated motif (PAM), follows immediately 3'- of a target motif in the target sequence.

In some embodiments, the Cas protein is introduced into the NK-92 cells in polypeptide form. In certain embodiments, the Cas proteins can be conjugated to or fused to a cell-penetrating polypeptide or cell-penetrating peptide that is well known in the art. Non-limiting examples of cell-penetrating peptides include those provided in Milletti F, "Cell-penetrating peptides: classes, orgin and current landscape." Drug Discov. Today 17: 850-860, 2012. In some cases, an unmodified NK-92 cell is genetically engineered to produce the Cas protein.

In some embodiments, the target motif in the target gene, to which the Cas protein is directed by the guide RNAs, is 17 to 23 bp in length. In some embodiments, the target motif is at least 20 bp in length. In some embodiments, the target motif is a 20-nucleotide DNA sequence. In some embodiments, the target motif is a 20-nucleotide DNA sequence and immediately precedes a short conserved sequence known as a protospacer-associated motif (PAM), recognized by the Cas protein. In some embodiments, the PAM motif is an NGG motif. In some embodiments, the target motif of the target gene is within an exon.

In some embodiments, the target motifs can be selected to minimize off-target effects of the CRISPR/Cas systems of the present invention. In some embodiments, the target motif is selected such that it contains at least two mismatches when compared with all other genomic nucleotide sequences in the cell. In some embodiments, the target motif is selected such that it contains at least one mismatch when compared with all other genomic nucleotide sequences in the cell. Those skilled in the art will appreciate that a variety of techniques can be used to select suitable target motifs for minimizing off-target effects (e.g., bioinformatics analyses).

The ribonucleic acids that are capable of directing the Cas protein to and hybridizing to a target motif in the target gene sequence are referred to as single guide RNA ("sgRNA"). The sgRNAs can be selected depending on the particular CRISPR/Cas system employed, and the sequence of the target polynucleotide, as will be appreciated by those skilled in the art. In some embodiments, the one to two ribonucleic acids can also be selected to minimize hybridization with nucleic acid sequences other than the target polynucleotide sequence. In some embodiments, the one to two ribonucleic acids hybridize to a target motif that contains at least two mismatches when compared with all other genomic nucleotide sequences in the cell. In some embodiments, the one to two ribonucleic acids hybridize to a target motif that contains at least one mismatch when compared with all other genomic nucleotide sequences in the cell. In some embodiments, the one to two ribonucleic acids are designed to hybridize to a target motif immediately adjacent to a deoxyribonucleic acid motif recognized by the Cas protein. In some embodiments, each of the one to two ribonucleic acids are designed to hybridize to target motifs immediately adjacent to deoxyribonucleic acid motifs recognized by the Cas protein which flank a mutant allele located between the target motifs. Guide RNAs can also be designed using software that are readily available, for example, at the websitecrispr.mit.edu. The one or more sgRNAs can be transfected into the NK-92 cells in which Cas protein is present by transfection, according to methods known in the art. In some embodiments, the sgRNAs that target TIGIT are one or more sgRNAs selected from the group consisting of SEQ ID NOs: 5-8.

Methods of using the CRISPR/Cas system to reduce gene expression are described in various publications, e.g., US. Pat. Pub. No. 2014/0170753.

### Zinc finger nuclease (ZFN)

In some embodiments, the modified NK-92 cells comprising a TIGIT-targeted alteration are produced by knocking out TIGIT in NK-92 cells using a zinc finger nuclease (ZFN). ZFNs are fusion proteins that comprise a non-specific cleavage domain (N) of FokI endonuclease and a zinc finger protein (ZFP). A pair of ZNFs are involved to recognize a specific locus in a target gene -- one that recognizes the sequence upstream and the other that recognizes the sequence downstream of the site to be modified-and the nuclease portion of the ZFN cuts at the specific locus and causing the knock out of the target TIGIT gene. Methods of using the ZFNs to reduce gene expression is well known, for example, as disclosed in U.S. Patent No. 9,045,763, and also in Durai et al., "Zinc Finger Nucleases: Custom-Designed Molecular Scissors for Genome Engineering of Plant and Mamalian cells," Nucleic Acid Research 33 (18):5978-5990 (2005).

### Transcription activator-like effector nucleases (TALENS)

In some embodiments, TIGIT-modified NK-92 cells comprising a targeted alteration are produced by knocking out TIGIT with transcription activator-like effector nucleases (TALENS). TALENs are similar to ZFNs in that they bind as a pair around a genomic site and direct the same non-specific nuclease, FoKI, to cleave the genome at a specific site, but instead of recognizing DNA triplets, each domain recognizes a single nucleotide. Methods of using the ZFNs to reduce gene expression are also well known, for example, as disclosed in U.S. Patent No. 9,005,973, and also Christian et al. "Targeting DNA Double-Strand Breaks with TAL Effector Nulceases," Genetics 186(2): 757-761 (2010).

### Knocking down TIGIT expression in NK-92 cells

In some embodiments, TIGIT-modified NK-92 cells comprising a targeted alteration are produced by knocking down one TIGIT with an interfering RNA. Interfering RNAs, when introduced *in vivo,* form an RNA-inducing silencing complex ("RISC") with other proteins and initiate a process known as RNA interference (RNAi). During the RNAi process, the RISC incorporates a single-stranded interfering RNA or one strand of a double stranded interfering RNA. The incorporated strand acts as a template for RISC to recognize complementary mRNA transcript. Once the complementary mRNA is identified, the protein components in RISC activate and cleave the mRNA, resulting in a knock-down of target gene expression. Non-limiting examples of interfering RNA molecules that be used to knock down expression of the target gene include siRNAs, short hairpin RNAs (shRNAs), single stranded interfering RNAs, and microRNAs (miRNAs). Methods for using these interfering RNAs are well known to one of skilled in the art.

In one embodiment, the interfering RNA is a siRNA. siRNA is a double stranded RNA which is typically less than 30 nucleotides long. Gene silencing by siRNA starts with one strand of the siRNA being incorporated into a ribonucleoprotein complex known as the RNA-induced silencing complex (RISC). The strand incorporated in RISC identifies mRNA molecules that are at least partially complementary to the incorporated siRNA strand and the RISC then cleaves these target mRNAs or inhibits their translation.

In one embodiment, the interfering RNA is a microRNA. microRNA is a small noncoding RNA molecule, which can hybridize to complementary sequences within mRNA molecules, resulting cleavage of the mRNA, or destabilization of the mRNA through shortening of its poly(A) tail.

In one embodiment, the interfering RNA is a single-stranded interfering RNA. The single strand can also effect mRNA silencing in a manner that is similar to the double stranded siRNA, albeit less efficient than, the double-stranded siRNA. The single-stranded interfering RNA typically has a length of about 19 to about 49 nucleotides as for the double-stranded siRNA described above.

A short hairpin RNA or small hairpin RNA (shRNA) is an artificial RNA molecule with a tight hairpin turn that can be used to silence target gene expression via the siRNA it produced in cells. Expression of shRNA in cells is typically accomplished by delivery of plasmids or through viral or bacterial vectors. Suitable vectors include but not limited to adeno-associated viruses (AAVs), adenoviruses, and lentiviruses. shRNA is an advantageous mediator of siRNA in that it has relatively low rate of degradation and turnover.

Interfering RNAs used herein may differ from naturally-occurring RNA by the addition, deletion, substitution or modification of one or more nucleotides. Non-nucleotide material may be bound to the interfering RNA, either at the 5' end, the 3' end, or internally. Non-limiting examples of modifications that interfering RNAs may contain relative to the naturally -occurring RNA are disclosed in U.S. Patent No. 8,399,653. Such modifications are commonly designed to increase the nuclease resistance of the interfering RNAs, to improve cellular uptake, to enhance cellular targeting, to assist in tracing the interfering RNA, to further improve stability, or to reduce the potential for activation of the interferon pathway. For example, interfering RNAs may comprise a purine nucleotide at the ends of overhangs. Conjugation of cholesterol to the 3' end of the sense strand of an siRNA molecule by means of a pyrrolidine linker, for example, also provides stability to an siRNA.

Interfering RNAs used herein are typically about 10-60, 10-50, or 10-40 (duplex) nucleotides in length, more typically about 8-15, 10-30, 10-25, or 10-25 (duplex) nucleotides in length, about 10-24, (duplex) nucleotides in length (e.g., each complementary sequence of the double-stranded siRNA is 10-60, 10-50, 10-40, 10-30, 10-25, or 10-25 nucleotides in length, about 10-24, 11-22, or 11-23 nucleotides in length, and the double-stranded siRNA is about 10-60, 10-50, 10-40, 10-30, 10-25, or 10-25 base pairs in length).

Techniques for selecting target motifs in a gene of interest for RNAi are known to those skilled in the art, for example, as disclosed in Tuschl, T. et al., "The siRNA User Guide," revised May 6, 2004, available on the Rockefeller University web site; by Technical Bulletin #506, "siRNA Design Guidelines," Ambion Inc. at Ambion's web site; and by other web-based design tools at, for example, the Invitrogen, Dharmacon, Integrated DNA Technologies, Genscript, or Proligo web sites. Initial search parameters can include G/C contents between 35% and 55% and siRNA lengths between 19 and 27 nucleotides. The target sequence may be located in the coding region or in the 5' or 3' untranslated regions of the mRNA. The target sequences can be used to derive interfering RNA molecules, such as those described herein.

Efficiency of the knock-out or knock-down can be assessed by measuring the amount of TIGIT mRNA or protein using methods well known in the art, for example, quantitative PCR, western blot, flow cytometry, etc and the like. In some embodiments, the level of TIGIT protein is evaluated to assess knock-out or knock-down efficiency. In certain embodiments, the efficiency of reduction of target gene expression is at least 5%, at least 10%, at least 20% , at least 30%, at least 50%, at least 60%, or at least 80%, or at least 90%, or greater, as compared to corresponding NK-92 cells that do not have the TIGIT-targeted alteration. In certain embodiments, the efficiency of reduction is from about 10% to about 90%. In certain embodiments, the efficiency of reduction is from about 30% to about 80%. In certain embodiments, the efficiency of reduction is from about 50% to about 80%. In some embodiments, the efficiency of reduction is greater than or equal to about 80%.

### NK-92 CELL MODIFIED TO DECREASE CD96 AND TIGIT EXPRESSION

In a further aspect, provided herein are NK-92 cells comprising a CD96-targeted alteration to reduce or eliminate CD96 expression and a TIGIT-targeted alteration to reduce or eliminate TIGIT expression. Such cells can be generated as described individually above for CD96-targeted or TIGIT-targeted alterations.

### NK-92 CELL MODIFIED TO DECREASE CD226 EXPRESSION

The disclosure further describes a modified NK-92 cell that is genetically modified to decrease CD226 expression, e.g., for use in comparative experiments as described in the examples section. In some embodiments, such a modified NK-92 cell comprises a CD226-targeted alteration to reduce or eliminate CD226 expression. Such cells can be generated using any of the techniques described hereinabove for modified NK-92 cells to decrease CD96 or TIGIT expression. Illustrative methods and cells produced using the methods are provided in the "Examples" section of the application.

### ADDITIONAL MODIFICATIONS

### Fc receptors

In some embodiments NK-92 cells comprising the CD96-targeted alteration and optional additional TIGIT-targeted alteration are further modified to express a Fc receptor on the cell surface. For example, in some embodiments, e.g., in which the further modified NK-92 cells are administered to a subject with a monoclonal antibody, the Fc receptor allows the NK cells to work in unison with antibodies that kill target cells through ADCC. In some embodiments, the Fc receptor is IgG Fc receptor FcγRIII.

Non-limiting examples of Fc receptors are provided below. These Fc receptors differ in their preferred ligand, affinity, expression, and effect following binding to the antibody.

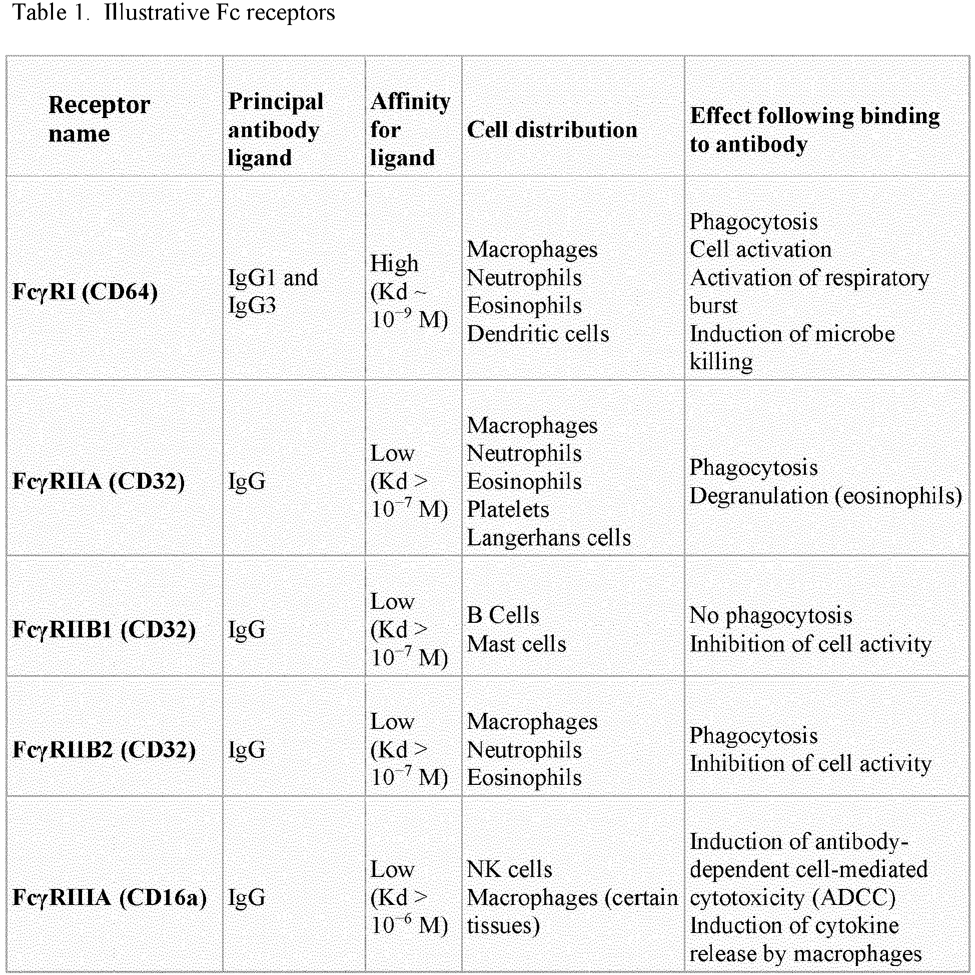

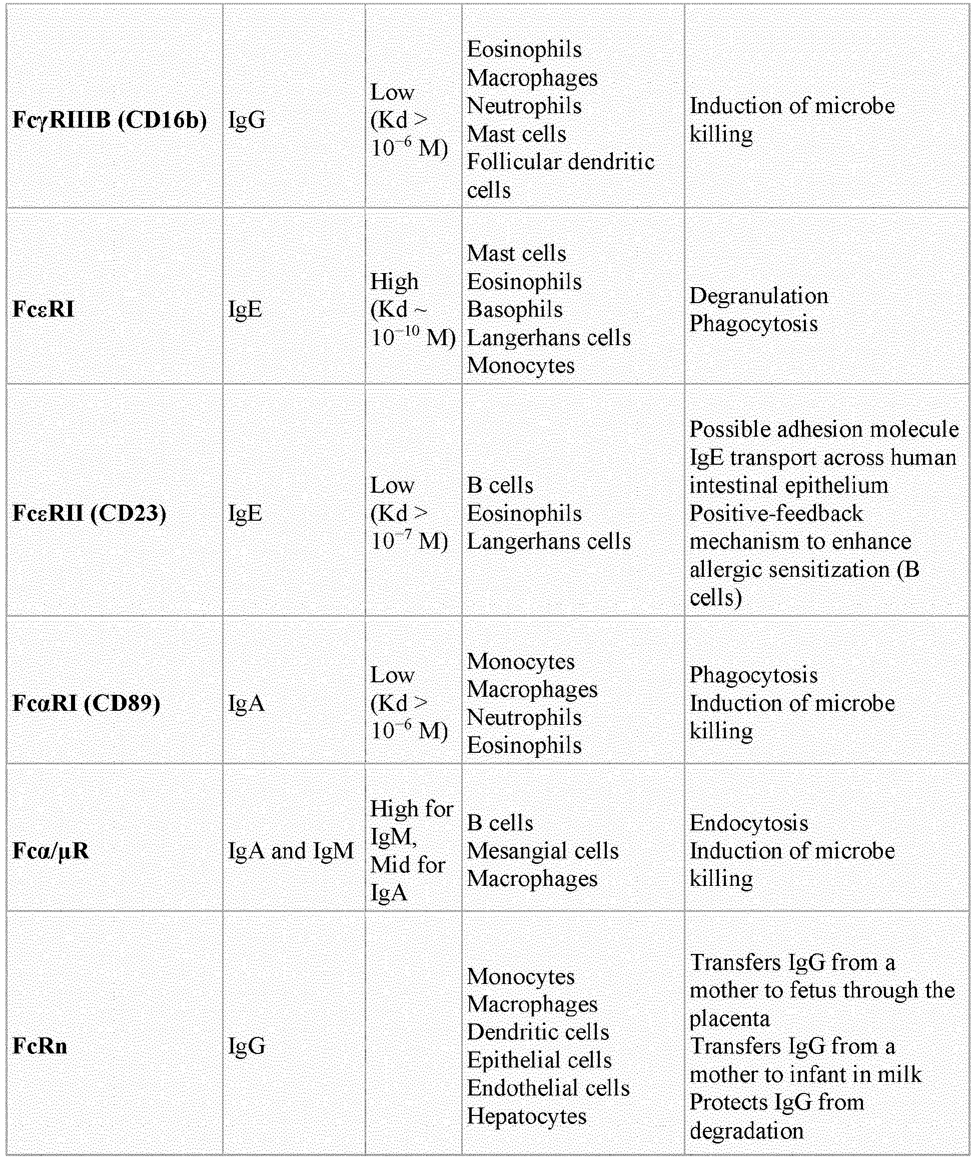

In some embodiments, the Fc receptor is CD16. In some embodiments, the Fc receptor is a high affinity form of CD16 in which a valine is present at position 176, numbered relative to the precursor form of the illustrative human CD16 polypeptide sequence provided in SEQ ID NO: 13. In some embodiments, the CD16 has at least 70%, at least 80%, at least 90%, or at least 95% identity to amino acids 19-254 of SEQ ID NO: 13 and comprises a valine at position 176, as numbered with reference to SEQ ID NO: 13.

### Chimeric Antigen Receptor

In some embodiments, the CD96-modified and optional additionally TIGIT-modified NK-92 cells are further engineered to express a chimeric antigen receptor (CAR) on the cell surface. Optionally, the CAR is specific for a tumor- specific antigen. Tumor-specific antigens are described, by way of non-limiting example, in US 2013/0189268; WO 1999024566 A1; US 7098008 ; and WO 2000020460 A1. Tumor-specific antigens include, without limitation, NKG2D, CS1, GD2, CD138, EpCAM, EBNA3C, GPA7, CD244, CA-125, ETA, MAGE, CAGE, BAGE, HAGE, LAGE, PAGE, NY-SEO-1, GAGE, CEA, CD52, CD30, MUC5AC, c-Met, EGFR, FAB, WT-1, PSMA, NY-ESO1, AFP, CEA, CTAG1B, CD19 and CD33. Additional non-limiting tumor-associated antigens, and the malignancies associated therewith, can be found in Table 2.

**Table 2: Tumor-Specific Antigens and Associated Malignancies**

| **Target Antigen** | **Associated Malignancy** |
|---|---|
| α-Folate Receptor | Ovarian Cancer |
| CAIX | Renal Cell Carcinoma |
| CD19 | B-cell Malignancies |
| | Chronic lymphocytic leukemia (CLL) |
| | B-cell CLL (B-CLL) |
| | Acute lymphoblastic leukemia (ALL); ALL post Hematopoietic stem cell transplantation (HSCT) |
| | Lymphoma; Refractory Follicular Lymphoma; B-cell non-Hodgkin lymphoma (B-NHL) |
| | Leukemia |
| | B-cell Malignancies post-HSCT |
| | B-lineage Lymphoid Malignancies post umbilical cord blood transplantation (UCBT) |
| CD19/CD20 | Lymphoblastic Leukemia |
| CD20 | Lymphomas |
| | B-Cell Malignancies |
| | B-cell Lymphomas |
| | Mantle Cell Lymphoma |
| | Indolent B-NHL |
| | Leukemia |
| CD22 | B-cell Malignancies |
| CD30 | Lymphomas; Hodgkin Lymphoma |
| CD33 | AML |
| CD44v7/8 | Cervical Carcinoma |
| CD138 | Multiple Myeloma |
| CD244 | Neuroblastoma |
| CEA | Breast Cancer |
| | Colorectal Cancer |
| CS1 | Multiple Myeloma |
| EBNA3C | EBV Positive T-cells |
| EGP-2 | Multiple Malignancies |
| EGP-40 | Colorectal Cancer |
| EpCAM | Breast Carcinoma |
| Erb-B2 | Colorectal Cancer |
| | Breast Cancer and Others |
| | Prostate Cancer |
| Erb-B 2,3,4 | Breast Cancer and Others |
| FBP | Ovarian Cancer |
| Fetal Acetylcholine Receptor | Rhabdomyosarcoma |
| GD2 | Neuroblastoma |
| GD3 | Melanoma |
| GPA7 | Melanoma |
| Her2 | Breast Carcinoma |
| | Ovarian Cancer |
| | Tumors of Epithelial Origin |
| Her2/new | Medulloblastoma |
| | Lung Malignancy |
| | Advanced Osteosarcoma |
| | Glioblastoma |
| IL-13R-a2 | Glioma |
| | Glioblastoma |
| | Medulloblastoma |
| KDR | Tumor Neovasculature |
| k-light chain | B-cell Malignancies |
| | B-NHL, CLL |
| LeY | Carcinomas |
| | Epithelial Derived Tumors |
| L1 Cell Adhesion Molecule | Neuroblastoma |
| MAGE-A1 | Melanoma |
| Mesothelin | Various Tumors |
| MUC1 | Breast Cancer; Ovarian Cancer |
| NKG2D Ligands | Various Tumors |
| Oncofetal Antigen (h5T4) | Various Tumors |
| PSCA | Prostate Carcinoma |
| PSMA | Prostate/Tumor Vasculature |
| TAA Targeted by mAb IgE | Various Tumors |
| TAG-72 | Adenocarcinomas |
| VEGF-R2 | Tumor Neovasculature |

In some embodiments, the CAR targets CD19, CD33 or CSPG-4. In some embodiments, the CAR targets an antigen associated with a specific cancer type. For example, the cancer may be selected from the group consisting of leukemia (including acute leukemias (e.g., acute lymphocytic leukemia, acute myelocytic leukemia (including myeloblastic, promyelocytic, myelomonocytic, monocytic, and erythroleukemia)) and chronic leukemias (e.g., chronic myelocytic (granulocytic) leukemia and chronic lymphocytic leukemia), polycythemia vera, lymphomas (e.g., Hodgkin's disease and non-Hodgkin's disease), multiple myeloma, Waldenstrom's macroglobulinemia, heavy chain disease, solid tumors including, but not limited to, sarcomas and carcinomas such as fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilm's tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, menangioma, melanoma, neuroblastoma and retinoblastoma.

CARs can be engineered as described, for example, in Patent Publication Nos. WO 2014039523; US 20140242701; US 20140274909; US 20130280285; and WO 2014099671. Optionally, the CAR is a CD19 CAR, a CD33 CAR or CSPG-4 CAR.

### Cytokines

In some embodiments, the invention provides CD96-modified and optionally further TIGIT-modified NK-92 cells that are further modified to express at least one cytokine. In such cells, the expression of cytokines in the cells may be directed to the endoplasmic reticulum. In some embodiments, the at least one cytokine is IL-2, IL-12, IL-15, IL-18, IL-21 or a variant thereof. In one embodiment, the cytokine is IL-2. In certain embodiments the IL-2 is a variant that is targeted to the endoplasmic reticulum.

In one embodiment, the IL-2 is expressed with a signal sequence that directs the IL-2 to the endoplasmic reticulum. Not to be bound by theory, but directing the IL-2 to the endoplasmic reticulum permits expression of IL-2 at levels sufficient for autocrine activation, but without releasing IL-2 extracellularly. See Konstantinidis et al "Targeting IL-2 to the endoplasmic reticulum confines autocrine growth stimulation to NK-92 cells" Exp Hematol. 2005 Feb;33(2):159-64.

In some embodiments, a suicide gene may also be inserted into CD96-modified and optionally also TIGIT-modified NK-92 cells to prevent unregulated growth of the cells. In some embodiments, the suicide gene is icaspase 9.

### Transgene expression

Also encompassed in the disclosure are sequences that share significant sequence identity to the polynucleotides or polypeptides described above, e.g., Cas proteins, CD16, Fc receptor, CAR, and/or IL-2. These sequences can also be introduced into the unmodified NK-92 cells. In some embodiments, the sequences have at least 70%, at least 80%, at least 85%, at least 88%, at least 95%, or at least 98%, or at least 99% sequence identity to their respective native sequences.

Transgenes (e.g. Cas proteins, CD16, Fc receptor, CAR, and/or IL-2) can be engineered into an expression plasmid by any mechanism known to those of skill in the art. Transgenes may be engineered into the same expression plasmid or different. In preferred embodiments, the transgenes are expressed on the same plasmid.

Transgenes can be introduced into NK-92 cells using any transient transfection method known in the art, including, for example, electroporation, lipofection, nucleofection, or "gene-gun."

Any number of vectors can be used to express these transgenes. In some embodiments, the vector is a retroviral vector. In some embodiments, the vector is a plasmid vector. Other viral vectors that can be used include adenoviral vectors, adeno-associated viral vectors, herpes simplex viral vectors, pox viral vectors, and others.

### Combination therapies

In some embodiments, CD96-modified and optionally also TIGIT-modified NK-92 cells of the present disclosure are used in combination with therapeutic antibodies and/or other anti-cancer agents. Therapeutic antibodies may be used to target cells that are infected or express cancer-associated markers. Examples of cancer therapeutic monoclonal antibodies are shown in Table 3.

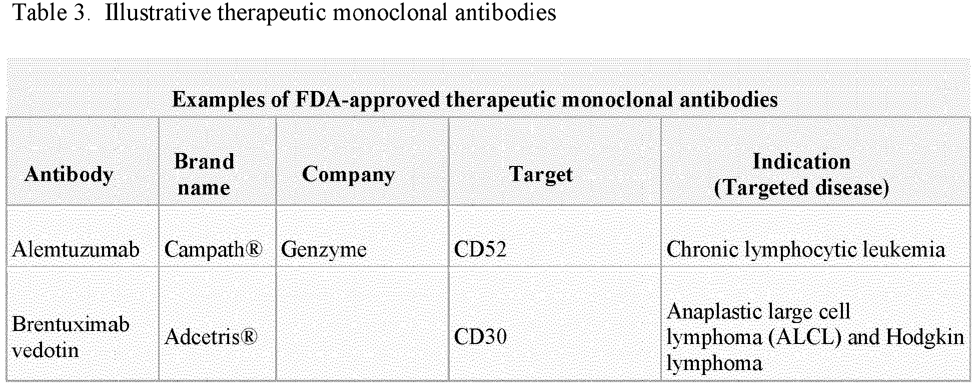

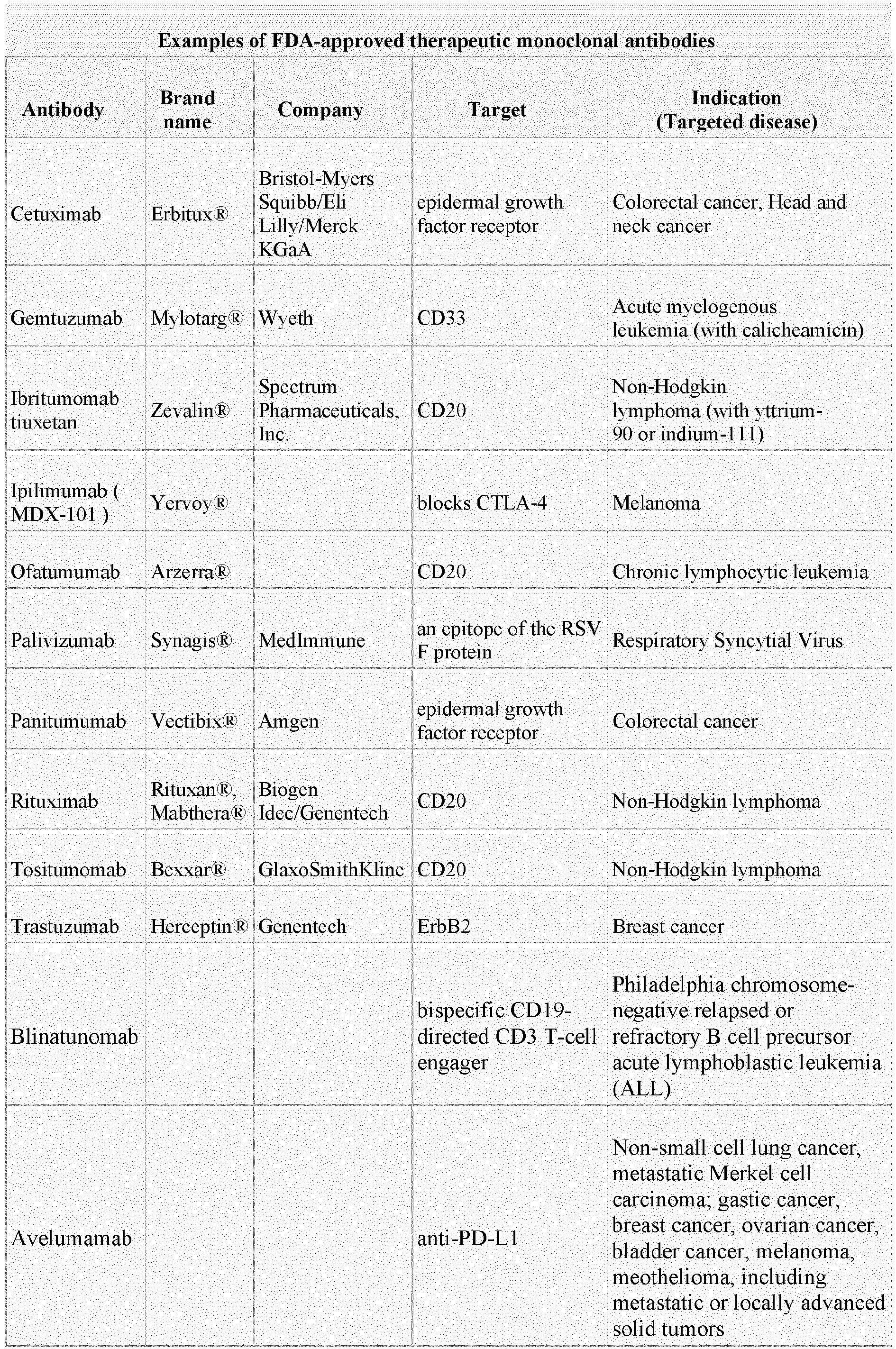

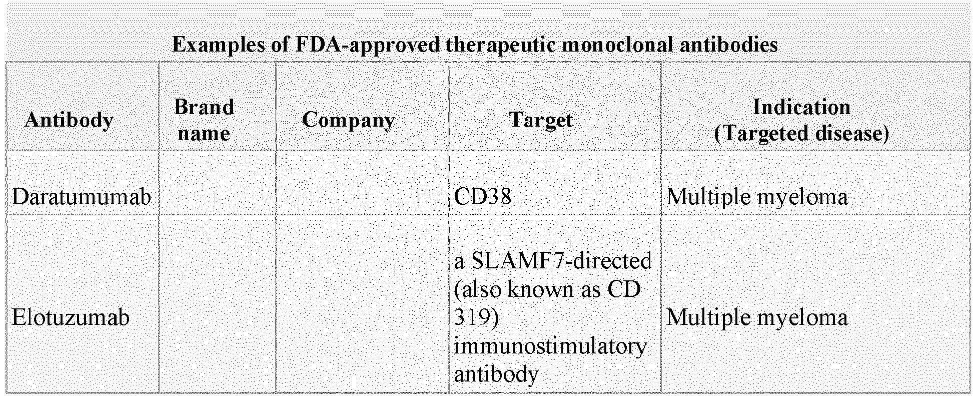

Antibodies may treat cancer through a number of mechanisms. Antibody-dependent cellular cytotoxicity (ADCC) occurs when immune cells, such as CD96-modified or TIGIT-modified NK cells of the present disclosure that also expresses FcR, bind to antibodies that are bound to target cells through Fc receptors, such as CD16. Accordingly, in some embodiments, CD96-modified or TIGIT-modified NK-92 cells expressing FcR are administered to a patient along with antibodies directed against a specific cancer-associated protein. Administration of such NK-92 cells may be carried out simultaneously with the administration of the monoclonal antibody, or in a sequential manner. In some embodiments, the NK-92 cells are administered to the subject after the subject has been treated with the monoclonal antibody. Alternatively, CD96-modified or TIGIT-modified NK-92 cells may be administered at the same time, e.g., within 24 hours, of the monoclonal antibody.

In some embodiments, CD96-modified (optionally plus TIGIT-modified) NK-92 cells are administered intravenously. In some embodiments such modified NK-92 cells may be infused directly into the bone marrow.

### Treatment

Also provided are methods of treating patients with CD96-modified and optionally also TIGIT-modified NK-92 cells as described herein. In some embodiments, the patient is suffering from cancer or an infectious disease. As described above, CD96-modified (optionally also TIGIT-modified) NK-92 cells may be further modified to express a CAR that targets an antigen expressed on the surface of the patient's cancer cells. In some embodiments, as explained above, CD96-modified optionally also TIGIT-modified NK-92 cells may also expressed and Fc receptor, *e.g.,* CD16. In further embodiments disclosed herein, the patient is treated with CD96-modified (optionally also TIGIT-modified) NK-92 cells and an antibody.

The modified NK-92 cells can be administered to an individual by absolute numbers of cells, e.g., said individual can be administered from about 1000 cells/injection to up to about 10 billion cells/injection, such as at about, at least about, or at most about, 1×10⁸, 1×10⁷, 5×10⁷, 1×10⁶, 5×10⁶, 1×10⁵, 5×10⁵, 1×10⁴, 5×10⁴, 1×10³, 5×10³ (and so forth) NK-92 cells per injection, or any ranges between any two of the numbers, end points inclusive.

In other embodiments, said individual can be administered from about 1000 cells/injection/m² to up to about 10 billion cells/injection/m², such as at about, at least about, or at most about, 1×10⁸/m², 1×10⁷/m², 5×10⁷/m², 1×10⁶/m², 5×10⁶/m², 1×10⁵/m², 5×10⁵/m², 1×10⁴/m², 5×10⁴/m², 1×10³/m², 5×10³/m² (and so forth) NK-92 cells per injection, or any ranges between any two of the numbers, end points inclusive.

In other embodiments, modified NK-92 cells can be administered to such individual by relative numbers of cells, e.g., said individual can be administered about 1000 cells to up to about 10 billion cells per kilogram of the individual, such as at about, at least about, or at most about, 1×10⁸, 1×10⁷, 5×10⁷, 1×10⁶, 5×10⁶, 1×10⁵, 5×10⁵, 1×10⁴, 5×10⁴, 1×10³, 5×10³ (and so forth) NK-92 cells per kilogram of the individual, or any ranges between any two of the numbers, end points inclusive.

In other embodiments, the total dose may be calculated by m² of body surface area, including about 1×10¹¹, 1×10¹⁰, 1×10⁹, 1×10⁸, 1×10⁷, per m², or any ranges between any two of the numbers, end points inclusive. The average person is about 1.6 to about 1.8 m². In a preferred embodiment, between about 1 billion and about 3 billion NK-92 cells are administered to a patient. In other embodiments, the amount of NK-92 cells injected per dose may calculated by m² of body surface area, including 1×10¹¹, 1×10¹⁰, 1×10⁹, 1×10⁸, 1×10⁷, per m². The average person is 1.6-1.8 m².

Modified NK-92 cells, and optionally other anti-cancer agents, can be administered once to a patient with cancer, or can be administered multiple times, e.g., once every 1, 2, 3, 4, 5, 6, 7, 8, 9, 10, 11, 12, 13, 14, 15, 16, 17, 18, 19, 20, 21, 22 or 23 hours, or once every 1, 2, 3, 4, 5, 6 or 7 days, or once every 1, 2, 3, 4, 5, 6, 7, 8, 9, 10 or more weeks during therapy, or any ranges between any two of the numbers, end points inclusive.

In some embodiments, CD96-modified NK-92 cells optionally also comprising TIGIT modification are administered in a composition comprising the modified NK-92 cells and a medium, such as human serum or an equivalent thereof. In some embodiments, the medium comprises human serum albumin. In some embodiments, the medium comprises human plasma. In some embodiments, the medium comprises about 1% to about 15% human serum or human serum equivalent. In some embodiments, the medium comprises about 1% to about 10% human serum or human serum equivalent. In some embodiments, the medium comprises about 1% to about 5% human serum or human serum equivalent. In a preferred embodiment, the medium comprises about 2.5% human serum or human serum equivalent. In some embodiments, the serum is human AB serum. In some embodiments, a serum substitute that is acceptable for use in human therapeutics is used instead of human serum. Such serum substitutes may be known in the art, or developed in the future. Although concentrations of human serum over 15% can be used, it is contemplated that concentrations greater than about 5% will be cost-prohibitive. In some embodiments, NK-92 cells are administered in a composition comprising NK-92 cells and an isotonic liquid solution that supports cell viability. In some embodiments, NK-92 cells are administered in a composition that has been reconstituted from a cryopreserved sample.

Pharmaceutically aceptable compositions can include a variety of carriers and excipients. A variety of aqueous carriers can be used, e.g., buffered saline and the like. These solutions are sterile and generally free of undesirable matter. Suitable carriers and excipients and their formulations are described in Remington: The Science and Practice of Pharmacy, 21st Edition, David B. Troy, ed., Lippicott Williams & Wilkins (2005). By pharmaceutically acceptable carrier is meant a material that is not biologically or otherwise undesirable, i.e., the material is administered to a subject without causing undesirable biological effects or interacting in a deleterious manner with the other components of the pharmaceutical composition in which it is contained. If administered to a subject, the carrier is optionally selected to minimize degradation of the active ingredient and to minimize adverse side effects in the subject. As used herein, the term pharmaceutically acceptable is used synonymously with physiologically acceptable and pharmacologically acceptable. A pharmaceutical composition will generally comprise agents for buffering and preservation in storage and can include buffers and carriers for appropriate delivery, depending on the route of administration.

These compositions for use in *in vivo* or *in vitro* may be sterilized by sterilization techniques employed for cells. The compositions may contain acceptable auxiliary substances as required to approximate physiological conditions such as pH adjusting and buffering agents, toxicity adjusting agents and the like, for example, sodium acetate, sodium chloride, potassium chloride, calcium chloride, sodium lactate and the like. The concentration of cells in these formulations and/or other agents can vary and will be selected primarily based on fluid volumes, viscosities, body weight and the like in accordance with the particular mode of administration selected and the subject's needs.

In one embodiment, CD96-modified and optionally also TIGIT-modified NK-92 cells are administered to the patient in conjunction with one or more other treatments for the cancer being treated. In some embodiments, two or more other treatments for the cancer being treated includes, for example, an antibody, radiation, chemotherapeutic, stem cell transplantation, or hormone therapy.

As explained above, in one embodiment, CD96-modified and optionally also TIGIT-modified NK-92 cells are administered in conjunction with an antibody targeting the diseased cells. In one embodiment, CD96-modified and optionally also TIGIT-modified NK-92 cells and an antibody are administered to the patient together, e.g., in the same formulation; separately, e.g., in separate formulations, concurrently; or can be administered separately, e.g., on different dosing schedules or at different times of the day. When administered separately, the antibody can be administered in any suitable route, such as intravenous or oral administration.

### Kits

Also disclosed are kits for the treatment of cancer or an infectious disease using compositions comprising an amount of CD96-modified and optionally also TIGIT-modified NK-92 cells as described herein. In some embodiments, the kits of the present disclosure may also include at least one monoclonal antibody.

In certain embodiments, the kit may contain additional compounds such as therapeutically active compounds or drugs that are to be administered before, at the same time or after administration of CD96-modified and optionally also TIGIT-modified cells. Examples of such compounds include an antibody, vitamins, minerals, fludrocortisone, ibuprofen, lidocaine, quinidine, chemotherapeutic, etc.

In various embodiments, instructions for use of the kits will include directions to use the kit components in the treatment of a cancer or an infectious disease. The instructions may further contain information regarding how to handle CD96-modified or TIGIT-modified NK-92 cells (e.g., thawing and/or culturing). The instructions may further include guidance regarding the dosage and frequency of administration.

Disclosed are materials, compositions, and components that can be used for, can be used in conjunction with, can be used in preparation for, or are products of the disclosed methods and compositions. These and other materials are disclosed herein, and it is understood that when combinations, subsets, interactions, groups, etc. of these materials are disclosed that while specific reference of each various individual and collective combinations and permutations of these compounds may not be explicitly disclosed, each is specifically contemplated and described herein. For example, if a method is disclosed and discussed and a number of modifications that can be made to a number of molecules including the method are discussed, each and every combination and permutation of the method, and the modifications that are possible are specifically contemplated unless specifically indicated to the contrary. Likewise, any subset or combination of these is also specifically contemplated and disclosed. This concept applies to all aspects of this disclosure including, but not limited to, steps in methods using the disclosed compositions. Thus, if there are a variety of additional steps that can be performed, it is understood that each of these additional steps can be performed with any specific method steps or combination of method steps of the disclosed methods, and that each such combination or subset of combinations is specifically contemplated and should be considered disclosed.

### EXAMPLES

The following examples are for illustrative purposes only and should not be interpreted as limitations of the claimed invention. There are a variety of alternative techniques and procedures available to those of skill in the art which would similarly permit one to successfully perform the intended invention.

### EXAMPLE 1.

### Materials and Methods

### Cell culture

NK-92 cells were maintained in X-VIVO 10 medium (Lonza, catalog # BE04-743Q) supplemented with 5% Human Serum (Valley Biomedical, catalog # HP1022) and recombinant human IL-2 (500 IU/ml; Prospec, catalog # Cyt-209). MCF-7, SKBR-3 and Daudi cell lines were purchased from American Type Culture Collection (ATCC, Rockville, MD), and maintained in RPMI-1640 medium (ThermoScientific, catalog # 61870-127) supplemented with 10% FBS (Gibco, catalog # 10438026) and 1% Penicillin/Streptomycin (Gibco, catalog # 15070-063).

### Generation of Cas9-NK-92 cells

NK-92 cells stably expressing Cas9 protein were generated by infecting NK-92 parental cells with the Edit-R Cas9 lentivirus. In brief, Edit-R Cas9 lentivirus stocks were produced by transfecting 7x10⁶ 293T cells per 10 cm petri dish with the following amount of plasmids: 7.5 µg Edit-R-Cas9 (Dharmacon, catalog # CAS10138), 5 µg pCMV-ΔR8.2, and 2.5 µg pCMV-VSV.G. The transfections were performed using Lipofectamine 3000 (Life Technologies, catalog # L3000-008) following manufacturer's instructions. Virus supernatants were collected 48 h post-transfection, and concentrated 10 fold using PEG-it Virus Precipitation Solution from System Biosciences (catalog # LV810A-1). 5x10⁵ NK-92 cells were infected by spinoculation (840 g for 99 min at 35°C) with 100 µl of concentrated virus in 1 ml of final medium in a 24 well plate, in the presence of TransDux (System Biosciences, catalog # LV850A-1). Forty eight hours post-transduction, the Cas9-expressing cells were selected by growing the cells in the presence of 15 µg/ml of blasticidin (InvivoGen, catalog # ant-bl-1).

### Generation of pT7-Guide-IVT CD96, TIGIT, and CD226 sgRNA constructs

The guide RNAs were designed using the MIT web tool http address crispr.mit.edu. Below are indicated the target sequences for each gene.

### CD96

The sgRNAs target the second exon of CD96 (NM_198196, transcript variant 1).

| **SEQ ID NO** | **Guide** | **Score** | **Sequence (5'→3')** | **PAM** | **Strand** | **Location in CD96 ORF** | **Number of off-target sites** |
|---|---|---|---|---|---|---|---|
| 1 | #1 | 90 | GCACAGTAGAAGCCGTATTG (SEQ ID NO:1) | GGG | minus | 220-239 | 74 (11 are in genes) |
| 2 | #2 | 89 | AGGCACAGTAGAAGCCGTAT (SEQ ID NO:2) | TGG | minus | 222-241 | 106 (10 are in genes) |
| 3 | #3 | 89 | GGCACAGTAGAAGCCGTATT (SEQ ID NO:3) | GGG | minus | 221-240 | 79 (10 are in genes) |
| 4 | #4 | 85 | ACGGCTTCTACTGTGCCTAT (SEQ ID NO:4) | GGG | plus | 224-243 | 74 (11 are in genes) |

### TIGIT

The sgRNAs target the second exon of TIGIT (NM_173799).

| **SEQ ID NO** | **Guide** | **Score** | **Sequence (5'→3')** | **PAM** | **Strand** | **Location in TIGIT ORF** | **Number of off-target sites** |
|---|---|---|---|---|---|---|---|
| 5 | #1 | 90 | TGGGGCCACTCGATCCTTGA (SEQ ID NO:5) | AGG | minus | 242-261 | 81 (11 are in genes) |
| 6 | #2 | 87 | CCCATCCTTCAAGGATCGAG (SEQ ID NO:6) | TGG | plus | 234-253 | 107 (15 are in genes) |
| 7 | #3 | 86 | CCACTCGATCCTTGAAGGAT (SEQ ID NO:7) | GGG | minus | 237-256 | 64 (11 are in genes) |
| 8 | #4 | 85 | GACCTGGGTCACTTGTGCCG (SEQ ID NO:8) | TGG | minus | 152-171 | 115 (20 are in genes) |

### CD226

The sgRNAs target the third exon of CD226 (NM_006566, transcript variant 1)

| **SEQ ID NO** | **Guide** | **Score** | **Sequence (5'→3')** | **PAM** | **Strand** | **Location in CD226 ORF** | **Number of off-target sites** |
|---|---|---|---|---|---|---|---|
| 9 | #1 | 86 | GTTCAAGATCGGGACCCAGC (SEQ ID NO:9) | AGG | plus | 150-169 | 53 (8 are in genes) |
| 10 | #2 | 85 | TAGAGACATGTTCTCGGCAA (SEQ ID NO:10) | AGG | minus | 86-105 | 116 (9 are in genes) |
| 11 | #3 | 80 | AGAGACATGTTCTCGGCAAA (SEQ ID NO:11) | GGG | minus | 85-104 | 186 10 are in genes) |
| 12 | #4 | 79 | AGGTGGAGTGGTTCAAGATC (SEQ ID NO: 12) | GGG | plus | 140-159 | 133 (25 are in genes) |

The target sites were cloned into the pT7-Guide-IVT plasmid (Origene, catalog # GE100025). The oligos were cloned using the two BsmBI sites in pT7-Guide-IVT, and following manufacturer's instructions. In vitro transcribed CD96, TIGIT, and CD226 sgRNAs were generated using the MEGAshortscript^{™} T7 Kit (Life Technologies, catalog # AM1354), following the manufacturer's instructions.

### Generation of CD96, TIGIT, CD226 single knock-out and CD96/TIGIT double knock-out NK-92 cells

*In vitro* transcribed sgRNAs were transfected into Cas9-NK-92 cells by electroporation, using the MaxCyte GT electroporator. Briefly, 5x10⁶ Cas9-NK-92 cells were transfected with 10 µg of *in vitro* transcribed sgRNA using NK-92-3-OC protocol. Initial experiments were performed to determine the most efficient sgRNA for each targeted gene. In these experiments sgRNAs 1 to 4 were transfected into Cas9-NK-92 cells, and the knock-out efficiency of each sgRNA was determined analyzing the expression of the targeted gene by flow cytometry at 48 hours post-transfection.

To generate CD96, TIGIT, and CD226 single knock-out NK-92 cells, Cas9-NK-92 cells were transfected with 10 µg of *in vitro* transcribed CD96 sgRNA-2, TIGIT sgRNA-2, and CD226 sgRNA-1 respectively. The double knock-out CD96/TIGIT NK-92 cells were generated by co-transfecting 10 µg of *in vitro* transcribed CD96 sgRNA-2 and TIGIT sgRNA-2. In all cases, the cells were plated by limited dilution 48 hours post-transfection. After growing the cells for 15 days, individual clones were selected, expanded and the expression of the targeted gene was determined by flow cytometry.

### Flow Cytometry

Cytofluorometric analysis of cell surface proteins was performed by direct immunostaining using the fluorophore-conjugated antibodies listed on the table below. Briefly, 10⁵ cells were stained with the recommended amount of antibody in 100 µl of flow cytometry staining buffer (PBS, 1% BSA) for 30 min, at 4°C, in the dark. Cells were washed twice with flow cytometry staining buffer, and resuspended in 200 µl of flow cytometry staining buffer. Samples were processed on a MACSQuant 10 flow cytometer (Miltenyi) and data was analyzed using FlowJo software.

| **Antibody** | **Vendor** | **Catalog #** |
|---|---|---|
| APC Mouse IgG1, κ Isotype Control | BD Biosciences | 555751 |
| Human CD96 v2 APC- conjugated | R&D Systems | Fab6199A |
| AF647 Mouse IgG1, κ Isotype Control | BD Pharmingen | 557714 |
| AF647 Mouse Anti-Human CD226 | BD Biosciences | 564797 |
| Anti-Human TIGIT PE conjugated | eBioscience | 12-9500-42 |
| mIgG1-PE Isotype | BioLegend | 400114 |
| APC anti-human CD155 (PVR) Antibody | BioLegend | 337618 |

### Cytotoxicity Assays

Target cells were stained with the fluorescent dye PKH67-GL (Sigma-Aldrich, Saint Louis, MO) according to manufacturer's instructions. Targets and effectors were combined at different effector to target ratios in a 96-well plate (Falcon BD, Franklin Lakes, NJ), briefly centrifuged, and incubated in X-VIVO 10 (Lonza, cat # 04-743Q) culture medium, supplemented with 5% human serum, at 37 °C for 4 h in a 5% CO2 incubator. After incubation, cells were stained with propidium iodide (PI, Sigma-Aldrich) at 10 µg/ml in 1% BSA/PBS buffer and analyzed immediately by flow cytometry. Dead target cells were identified as double positive for PKH67-GL and PI. Target cells and effector cells were also stained separately with PI to assess spontaneous cell lysis. The percentage of NK-mediated cytotoxicity was obtained by subtracting the percentage of PKH(+)/PI(+) cells for target cells alone (spontaneous lysis) from the percentage of PKH(+)/PI(+) cells in the samples with effectors

### Results

### Generation of CD96, TIGIT, CD226 single knock-out and CD96/TIGIT double knock-out NK-92 cells

The NK-92 cell line is a Natural Killer-like cell line that was established from the peripheral blood of a 50 year old male Caucasian patient with Non-Hodgkin's Lymphoma (Gong et al., Leukemia 8:652-8, 1994). NK-92 cells are positive for CD2, CD56 and CD57, and negative for CD3 and CD16 (see, Gong *et al*.,). Their growth is IL-2-dependent and they exert potent in vitro cytotoxicity against a broad range of tumor targets. NK-92 cells lack most of currently known inhibitory KIR receptors (Maki *et al., supra*)*.* However, they express CD226, CD96, and TIGIT (Figure 1), which are members of a family of receptors that bind nectin and nectin-like proteins, and that have crucial roles in regulating NK cell function. CD226, also known as DNAM1, is an activating receptor critical for mediating NK cell cytotoxicity (Shibuya *et al., supra*)*,* while CD96 and TIGIT have been shown to act as inhibitory immune checkpoints to dampen NK functional activity (Chan et al., Nat Immunol. 15:431-8, 2014; Sarhan et al., Cancer Res. 76:5696-5706, 2016). NK-92 variants were generated that lack one or more of these inhibitory receptors in order to increase their anti-tumor potential.

The CRISPR/Cas9 system was used to generate NK-92 cells lacking expression of CD96, TIGIT or CD226. Single CD96, TIGIT, and CD226, or double CD96/TIGIT knock-out NK-92 cells were generated as described above in "Materials and Methods". Figure 2 shows the expression of these receptors in selected single or double KO clones. Of note, the expression of the non-targeted receptors on the single or double KO cells was comparable to that of parental NK-92 cells (see MFI values on Figure 2).

### CD96 and CD96/TIGIT knock-out NK-92 cells have higher cytotoxicity potential against CD155-positive tumor targets

The activating CD226 and the inhibitory CD96 and TIGIT receptors share a common ligand, CD155 (also known as PVR, polio virus receptor), to which they bind with different affinities (Martinet *et al., supra*)*.* CD155 expression is frequently upregulated in tumor cells, and its over-expression is associated with cancer invasiveness and metastasis (Hirota *et al.,* Sloan *et al.,* both *supra*)*.* Therefore, the cytotoxicity ability of parental and nectin-receptor knock-out cells against CD155-positive tumor targets was first tested. MCF-7 and SKBR-3 are two breast cancer cell lines that are positive for CD155 expression (Figure 3). Consistent with the role of CD226 as an activating receptor, killing of MCF-7 or SKBR-3 by CD226-KO NK-92 cells was almost completely abrogated (Figure 4).

Importantly, CD96-KO NK-92 cells have a 10-15% higher cytotoxic activity as compared to parental NK-92 cells (Figure 4). Although TIGIT-KO NK-92 cells did not differ significantly from parental NK-92 cells in their ability to kill MCF-7 or SKBR-3 cells, the double CD96/TIGIT KO cells had a higher cytotoxicity potential than that of CD96-KO or parental NK-92 cells (10-15% and 17-29% higher cytotoxic activity than CD96-KO or parental NK-92 cells respectively, as shown in Figure 4). These data thus indicate that although CD96 might compensate for the lack of TIGIT expression, both receptors are actively contributing to dampening the activatory signals mediated by CD226 and required for efficient killing of tumor targets.

Importantly, the higher cytotoxicity activity of CD96 and CD96/TIGIT KO NK-92 cells against CD155-positive tumor targets is specific to the loss of the nectin receptors, and not to intrinsic higher cytotoxic activity of these clones, since their cytotoxic activity against CD155-negative Daudi tumor cells (Figure 3) did not differ significantly from that of parental NK-92 cells (Figure 5). Of note, CD226-KO NK-92 cells killed Daudi cells less efficiently at the lowest E:T ratios, which suggests that Daudi cells might express additional ligands, other than CD155, which are also recognized by the activating receptor CD226 (Figure 5).

**Illustrative CD 16 sequence: SEQ ID NO:13 High Affinity Variant Immunoglobulin Gamma Fc Region Receptor III-A amino acid sequence (precursor form).** Position 176 of the precursor form corresponds to position 158 of a mature form of the polypeptide that starts with the Arg at position 19. The Val at position 176 is underlined.

Met Trp Gln Leu Leu Leu Pro Thr Ala Leu Leu Leu Leu Val Ser Ala Gly Met Arg Thr Glu Asp Leu Pro Lys Ala Val Val Phe Leu Glu Pro Gln Trp Tyr Arg Val Leu Glu Lys Asp Ser Val Thr Leu Lys Cys Gln Gly Ala Tyr Ser Pro Glu Asp Asn Ser Thr Gln Trp Phe His Asn Glu Ser Leu Ile Ser Ser Gln Ala Ser Ser Tyr Phe Ile Asp Ala Ala Thr Val Asp Asp Ser Gly Glu Tyr Arg Cys Gln Thr Asn Leu Ser Thr Leu Ser Asp Pro Val Gln Leu Glu Val His Ile Gly Trp Leu Leu Leu Gln Ala Pro Arg Trp Val Phe Lys Glu Glu Asp Pro Ile His Leu Arg Cys His Ser Trp Lys Asn Thr Ala Leu His Lys Val Thr Tyr Leu Gln Asn Gly Lys Gly Arg Lys Tyr Phe His His Asn Ser Asp Phe Tyr Ile Pro Lys Ala Thr Leu Lys Asp Ser Gly Ser Tyr Phe Cys Arg Gly Leu Val Gly Ser Lys Asn Val Ser Ser Glu Thr Val Asn Ile Thr Ile Thr Gln Gly Leu Ala Val Ser Thr Ile Ser Ser Phe Phe Pro Pro Gly Tyr Gln Val Ser Phe Cys Leu Val Met Val Leu Leu Phe Ala Val Asp Thr Gly Leu Tyr Phe Ser Val Lys Thr Asn Ile Arg Ser Ser Thr Arg Asp Trp Lys Asp His Lys Phe Lys Trp Arg Lys Asp Pro Gln Asp Lys

## Claims

1. A modified NK-92 cell comprising one or more alterations that inhibit expression of one or more target genes, wherein CD96 is one of the one or more target genes.

2. The modified NK-92 cell according to claim 1, wherein the target genes are CD96 and TIGIT.

3. The modified NK-92 cell of claim 1 or claim 2, in which the CD96 gene is genetically altered to inhibit expression of CD96.

4. The modified NK-92 cell according to claim 3, wherein :
the modified NK-92 cell comprises an interfering RNA that targets CD96 and inhibits expression of CD96; or
the cell is produced by knocking down or knocking out CD96 expression in the cell.

5. The modified NK-92 cell of claim 2, in which the TIGIT gene is genetically altered to inhibit expression of TIGIT.

6. The modified NK-92 cell of claim 5, wherein:
the modified NK-92 cell comprises an interfering RNA that targets TIGIT and inhibits expression of TIGIT; or
the cell is produced by knocking down or knocking out TIGIT expression in the cell.

7. The modified NK-92 cell of claim 2, wherein:
the modified NK-92 cells comprises an interfering RNA that targets CD96 and inhibits expression of CD96; and an interfering RNA that targets TIGIT and inhibits expression of TIGIT; or
the modified NK-92 cell is produced by knocking down or knocking out CD96 expression and knocking down or knocking out TIGIT expression in the cell.

8. The modified NK-92 cell of any one of claims 1 to 7, wherein the modified NK cell expresses at least one Fc receptor, or at least one chimeric antigen receptor (CAR), or both at least one Fc receptor and at least one CAR on the cell surface; optionally (i) wherein the at least one Fc receptor is CD16 or a CD16 polypeptide having a valine at position 176, as numbered with reference to SEQ ID NO:13 and wherein the at least one Fc receptor may optionally comprise a polynucleotide sequence encoding a polypeptide having at least 90% sequence identity to amino acids 19-254 of SEQ ID NO:13 and comprises a valine at position 176 as numbered with reference to SEQ ID NO:13; or
(ii) the at least one Fc receptor is FcyRIII; or
(iii) the CAR comprises a cytoplasmic domain of FcεRIγ, optionally wherein the CAR targets a tumor-associated antigen.

9. The modified NK-92 cell of any one of claims 1 to 8, wherein the cell further expresses a cytokine, optionally wherein the cytokine is interleukin-2 or a variant thereof and/or wherein the cytokine is targeted to the endoplasmic reticulum.

10. A composition comprising a plurality of modified NK-92 cells of any preceding claim, optionally (i) wherein the composition further comprises a physiologically acceptable excipient.

11. An NK-92 cell line comprising a plurality of modified NK-92 cells of any one of clams 1 to 9.

12. The cell line of claim 11 wherein the cells undergo less than 10 population doublings.

13. A therapeutically effective amount of the composition of claim 10(i) or the cell line of claim 11 or 12, for use in a method of treating cancer in a subject in need thereof,
optionally wherein the method further comprises administering an antibody; and/or
wherein about 1x10⁸ to about 1x10¹¹ cells per m² of body surface area of the patient are administered to the patient.

14. A kit for treating cancer, wherein the kit comprises (a) a composition of claim 10; or a cell line of claim 11 or 12.

15. A method for producing an NK-92 cell that expresses decreased levels of one or more target genes relative to a control NK-92 cell, the method comprising genetically modifying the expression of the one or more target genes in the NK-92 cell, wherein CD96 is one of the one or more target genes, optionally wherein the one or more target genes consist of CD96 and TIGIT.

16. The method of claim 15, wherein:
(a) the step of genetically modifying the expression of each of the one or more target genes comprises contacting a NK-92 cell to be modified with an interfering RNA targeting each of the one or more target genes, optionally wherein the interfering RNA targeting each of the one or more target genes is an siRNA, an shRNA, a microRNA, or a single stranded interfering RNA; or
(b) the step of genetically modifying the expression of each of the one or more target genes comprises modifying the each of the one or more target genes with a zinc finger nuclease (ZFN), a Tale-effector domain nuclease (TALEN), or a CRIPSR/Cas system; or
(c) genetically modifying the expression of each of the one or more target genes comprises:
i) introducing a clustered regularly interspaced short palindromic repeat-associated (Cas) protein into the NK-92 cell and
ii) introducing one or more ribonucleic acids in the NK-92 cell to be modified, wherein the ribonucleic acids direct the Cas protein to hybridize to a target motif of the sequence of the each of the one or more target genes, and wherein the target motif is cleaved, optionally wherein:
the Cas protein is introduced into the NK-92 cell in protein form, or
the Cas protein is introduced into the NK-92 cell by introducing a Cas coding sequence, or
the Cas protein is Cas9, or
the target motif is a 20 nucleotide DNA sequence, or
the target motif is in an exon of each of the one or more target genes, or
the one or more ribonucleic acids that hybridize to a target motif in CD96 are selected from the group consisting of SEQ ID NO: 1-4 and the one or more ribonucleic acids that hybridize to a target motif in TIGIT are selected from the group consisting of SEQ ID NOs:5-8.

## Patentansprüche

1. Modifizierte NK-92-Zelle, eine oder mehrere Veränderungen umfassend, die eine Expression eines oder mehrerer Zielgene hemmen, wobei CD96 eines von dem einen oder den mehreren Zielgenen ist.

2. Modifizierte NK-92-Zelle nach Anspruch 1, wobei die Zielgene CD96 und TIGIT sind.

3. Modifizierte NK-92-Zelle nach Anspruch 1 oder Anspruch 2, in der das CD96-Gen genetisch so verändert ist, dass es eine Expression von CD96 hemmt.

4. Modifizierte NK-92-Zelle nach Anspruch 3, wobei:
die modifizierte NK-92-Zelle eine interferierende RNA, die auf CD96 abzielt und eine Expression von CD96 hemmt, umfasst; oder
die Zelle durch Mindern oder Abschalten der CD96-Expression in der Zelle erzeugt wird.

5. Modifizierte NK-92-Zelle nach Anspruch 2, in der das TIGIT-Gen genetisch so verändert ist, dass es eine Expression von TIGIT hemmt.

6. Modifizierte NK-92-Zelle nach Anspruch 5, wobei:
die modifizierte NK-92-Zelle eine interferierende RNA, die auf TIGIT abzielt und eine Expression von TIGIT hemmt, umfasst; oder
die Zelle durch Mindern oder Abschalten der TIGIT-Expression in der Zelle erzeugt wird.

7. Modifizierte NK-92-Zelle nach Anspruch 2, wobei:
die modifizierten NK-92-Zellen eine interferierende RNA, die auf CD96 abzielt und eine Expression von CD96 hemmt; und eine interferierende RNA, die auf TIGIT abzielt und eine Expression von TIGIT hemmt, umfassen; oder
die modifizierte NK-92-Zelle durch Mindern oder Abschalten der CD96-Expression und durch Mindern oder Abschalten der TIGIT-Expression in der Zelle erzeugt wird.

8. Modifizierte NK-92-Zelle nach einem der Ansprüche 1 bis 7, wobei die modifizierte NK-Zelle mindestens einen Fc-Rezeptor oder mindestens einen chimären Antigenrezeptor (CAR) oder sowohl mindestens einen Fc-Rezeptor als auch mindestens einen CAR auf der Zelloberfläche exprimiert; optional (i) wobei der mindestens eine Fc-Rezeptor CD16 oder ein CD16-Polypeptid mit einem Valin an Position 176 ist, wie mit Bezug auf SEQ ID NO: 13 nummeriert, und wobei der mindestens eine Fc-Rezeptor optional eine Polynukleotidsequenz umfassen kann, die ein Polypeptid codiert, das eine Sequenzidentität von mindestens 90 % mit den Aminosäuren 19-254 von SEQ ID NO: 13 aufweist und ein Valin an Position 176, wie mit Bezug auf SEQ ID NO: 13 nummeriert, umfasst; oder
(ii) der mindestens eine Fc-Rezeptor FcγRIII ist; oder
(iii) der CAR eine cytoplasmatische Domäne von FcεRIγ umfasst, wobei der CAR optional auf ein tumorassoziiertes Antigen abzielt.

9. Modifizierte NK-92-Zelle nach einem der Ansprüche 1 bis 8, wobei die Zelle ferner ein Zytokin exprimiert, wobei das Zytokin optional Interleukin-2 oder eine Variante davon ist und/oder wobei das Zytokin auf das endoplasmatische Retikulum abzielt.

10. Zusammensetzung, eine Vielzahl modifizierter NK-92-Zellen nach einem vorhergehenden Anspruch umfassend, optional (i) wobei die Zusammensetzung ferner einen physiologisch unbedenklichen Hilfsstoff umfasst.

11. NK-92-Zelllinie, eine Vielzahl modifizierter NK-92-Zellen nach einem der Ansprüche 1 bis 9 umfassend.

12. Zelllinie nach Anspruch 11, wobei die Zellen weniger als 10 Populationsverdoppelungen erfahren.

13. Therapeutisch wirksame Menge der Zusammensetzung nach Anspruch 10(i) oder der Zelllinie nach Anspruch 11 oder 12 zur Verwendung in einem Verfahren zum Behandeln von Krebs bei einem Subjekt, das dies benötigt,
wobei das Verfahren optional ferner Verabreichen eines Antikörpers umfasst; und/oder wobei dem Patienten etwa 1x10⁸ bis etwa 1x10¹¹ Zellen pro m² Körperoberfläche des Patienten verabreicht werden.

14. Kit zum Behandeln von Krebs, wobei das Kit (a) eine Zusammensetzung nach Anspruch 10; oder eine Zelllinie nach Anspruch 11 oder 12 umfasst.

15. Verfahren zum Erzeugen einer NK-92-Zelle, die im Vergleich zu einer Kontroll-NK-92-Zelle verringerte Niveaus eines oder mehrerer Zielgene exprimiert, wobei das Verfahren genetisches Modifizieren der Expression des einen oder der mehreren Zielgene in der NK-92-Zelle umfasst, wobei CD96 eines von dem einen oder den mehreren Zielgenen ist, wobei das eine oder die mehreren Zielgene optional aus CD96 und TIGIT bestehen.

16. Verfahren nach Anspruch 15, wobei:
(a) der Schritt des genetischen Modifizierens der Expression jedes von dem einen oder den mehreren Zielgenen Inkontaktbringen einer zu modifizierenden NK-92-Zelle mit einer interferierenden RNA, die auf jedes von dem einen oder den mehreren Zielgenen abzielt, umfasst, wobei die interferierende RNA, die auf jedes von dem einen oder den mehreren Zielgenen abzielt, optional eine siRNA, eine shRNA, eine microRNA oder eine einzelsträngige interferierende RNA ist; oder
(b) der Schritt des genetischen Modifizierens der Expression jedes von dem einen oder den mehreren Zielgenen Modifizieren des jeden von dem einen oder den mehreren Zielgenen mit einer Zinkfingernuklease (ZFN), einer Tale-Effektordomänennuklease (TALEN) oder einem CRIPSR/Cas-System umfasst; oder
(c) das genetische Modifizieren der Expression jedes von dem einen oder den mehreren Zielgenen Folgendes umfasst:
i) Einbringen eines Clustered-Regularly-Interspaced-Short-Palindromic-Repeat-assoziierten (Cas)-Proteins in die NK-92-Zelle und
ii) Einführen einer oder mehrerer Ribonukleinsäuren in die zu modifizierende NK-92-Zelle, wobei die Ribonukleinsäuren das Cas-Protein anweisen, mit einem Zielmotiv der Sequenz des jeden von dem einen oder den mehreren Zielgenen zu hybridisieren, und wobei das Zielmotiv gespalten wird, optional wobei:
das Cas-Protein in Proteinform in die NK-92-Zelle eingeführt wird, oder
das Cas-Protein in die NK-92-Zelle durch Einführen einer Cas-Codierungssequenz eingeführt wird, oder
das Cas-Protein Cas9 ist, oder
das Zielmotiv eine 20-Nukleotiden-DNA-Sequenz ist, oder
das Zielmotiv sich in einem Exon jedes von dem einen oder den mehreren Zielgenen befindet, oder
die eine oder mehreren Ribonukleinsäuren, die mit einem Zielmotiv in CD96 hybridisieren, aus der Gruppe ausgewählt sind, die aus SEQ ID NO: 1-4 besteht, und die eine oder mehreren Ribonukleinsäuren, die mit einem Zielmotiv in TIGIT hybridisieren, aus der Gruppe ausgewählt sind, die aus SEQ ID NO: 5-8 besteht.

## Revendications

1. Cellule NK-92 modifiée comprenant une ou plusieurs altérations qui inhibent l'expression d'un ou plusieurs gènes cibles, dans laquelle CD96 est l'un du ou des gènes cibles.

2. Cellule NK-92 modifiée de la revendication 1, dans laquelle les gènes cibles sont CD96 et TIGIT.

3. Cellule NK-92 modifiée de la revendication 1 ou la revendication 2, dans laquelle le gène CD96 est génétiquement altéré pour inhiber l'expression de CD96.

4. Cellule NK-92 modifiée de la revendication 3, dans laquelle :
la cellule NK-92 modifiée comprend un ARN interférent qui cible CD96 et inhibe l'expression de CD96 ; ou
la cellule est produite en réduisant ou en éliminant l'expression de CD96 dans la cellule.

5. Cellule NK-92 modifiée de la revendication 2, dans laquelle le gène TIGIT est génétiquement altéré pour inhiber l'expression de TIGIT.

6. Cellule NK-92 modifiée de la revendication 5, dans laquelle :
la cellule NK-92 modifiée comprend un ARN interférent qui cible TIGIT et inhibe l'expression de TIGIT ; ou
la cellule est produite en réduisant ou en éliminant l'expression de TIGIT dans la cellule.

7. Cellule NK-92 modifiée de la revendication 2, dans laquelle :
les cellules NK-92 modifiées comprennent un ARN interférent qui cible CD96 et inhibe l'expression de CD96 ; et un ARN interférent qui cible TIGIT et inhibe l'expression de TIGIT ; ou
la cellule NK-92 modifiée est produite en réduisant ou en éliminant l'expression de CD96 et en réduisant ou en éliminant l'expression de TIGIT dans la cellule.

8. Cellule NK-92 modifiée de l'une quelconque des revendications 1 à 7, dans laquelle la cellule NK modifiée exprime au moins un récepteur Fc, ou au moins un récepteur d'antigène chimérique (CAR), ou à la fois au moins un récepteur Fc et au moins un CAR sur la surface cellulaire ; éventuellement (i) dans laquelle l'au moins un récepteur Fc est CD16 ou un polypeptide CD16 comportant une valine en position 176, telle que numérotée en référence à SEQ ID N° : 13 et dans laquelle l'au moins un récepteur Fc peut éventuellement comprendre une séquence polynucléotidique codant pour un polypeptide comportant au moins 90 % d'identité de séquence avec les acides aminés 19 à 254 de SEQ ID N° : 13 et comprend une valine à la position 176 telle que numérotée en référence à SEQ ID N° : 13 ; ou
(ii) l'au moins un récepteur Fc est FcyRIII ; ou
(iii) le CAR comprend un domaine cytoplasmique de FcεRIγ, éventuellement dans laquelle le CAR cible un antigène associé à une tumeur.

9. Cellule NK-92 modifiée de l'une quelconque des revendications 1 à 8, dans laquelle la cellule exprime en outre une cytokine, éventuellement dans laquelle la cytokine est l'interleukine-2 ou un variant de celle-ci, et/ou dans laquelle la cytokine est ciblée sur le réticulum endoplasmique.

10. Composition comprenant une pluralité de cellules NK-92 modifiées de l'une quelconque des revendications précédentes, éventuellement (i) dans laquelle la composition comprend en outre un excipient physiologiquement acceptable.

11. Lignée cellulaire NK-92 comprenant une pluralité de cellules NK-92 modifiées de l'une quelconque des revendications 1 à 9.

12. Lignée cellulaire de la revendication 11, dans laquelle les cellules subissent moins de 10 doublements de population.

13. Quantité thérapeutiquement efficace de la composition de la revendication 10 (i) ou de la lignée cellulaire de la revendication 11 ou 12, destinée à être utilisée dans un procédé de traitement du cancer chez un sujet en ayant besoin,
éventuellement dans laquelle le procédé comprend en outre l'administration d'un anticorps ; et/ou
dans laquelle environ 1×10⁸ à environ 1×10¹¹ de cellules par m² de surface corporelle du patient sont administrées au patient.

14. Kit pour le traitement du cancer, dans lequel le kit comprend (a) une composition de la revendication 10 ; ou une lignée cellulaire de la revendication 11 ou 12.

15. Procédé de production d'une cellule NK-92 qui exprime des niveaux réduits d'un ou de plusieurs gènes cibles par rapport à une cellule NK-92 témoin, le procédé comprenant la modification génétique de l'expression du ou des gènes cibles dans la cellule NK-92, dans lequel CD96 est l'un du ou des gènes cibles, éventuellement dans lequel le ou les gènes cibles sont constitués de CD96 et TIGIT.

16. Procédé de la revendication 15, dans lequel :
(a) l'étape de modification génétique de l'expression de chacun du ou des gènes cibles comprend la mise en contact d'une cellule NK-92 à modifier avec un ARN interférent ciblant chacun du ou des gènes cibles, éventuellement dans lequel l'ARN interférent ciblant chacun du ou des gènes cibles est un siRNA, un shRNA, un microARN ou un ARN interférent simple brin ; ou
(b) l'étape de modification génétique de l'expression de chacun du ou des gènes cibles comprend la modification de chacun du ou des gènes cibles avec une nucléase à doigt de zinc (ZFN), une nucléase à domaine effecteur Tale (TALEN) ou un système CRIPSR/Cas ; ou
(c) la modification génétique de l'expression de chacun du ou des gènes cibles comprend :
i) l'introduction d'une protéine associée à des répétitions palindromiques courtes regroupées et régulièrement espacées (Cas) dans la cellule NK-92 et
ii) l'introduction d'un ou plusieurs acides ribonucléiques dans la cellule NK-92 à modifier, les acides ribonucléiques dirigeant la protéine Cas pour s'hybrider à un motif cible de la séquence de chacun du ou des gènes cibles, et le motif cible étant clivé, éventuellement dans lequel :
la protéine Cas est introduite dans la cellule NK-92 sous forme de protéine, ou
la protéine Cas est introduite dans la cellule NK-92 en introduisant une séquence codante Cas, ou
la protéine Cas est Cas9, ou
le motif cible est une séquence d'ADN de 20 nucléotides, ou
le motif cible se trouve dans un exon de chacun du ou des gènes cibles, ou
le ou les acides ribonucléiques qui s'hybrident à un motif cible dans CD96 sont sélectionnés dans le groupe constitué de SEQ ID N° : 1 à 4 et le ou les acides ribonucléiques qui s'hybrident à un motif cible dans TIGIT sont sélectionnés dans le groupe constitué de SEQ ID N° : 5 à 8.
